# FASCICULE DE BREVET EUROPEEN

(11) **EP 0 682 713 B1**
(45) Date de publication et mention de la délivrance du brevet: **15.09.1999**
(21) Numéro de dépôt: 94907582.4
(22) Date de dépôt: 15.02.1994
(51) Int. Cl.: C12P 19/04, C12P 19/26

(54) **BACTERIES DU TYPE ALTEROMONAS, POLYSACCHARIDES PRODUITS PAR CES BACTERIES, OSE CONTENU DANS CES POLYSACCHARIDES ET APPLICATIONS**
BAKTERIEN VOM ALTEROMONASTYP, DURCH DIESE BAKTERIEN HERGESTELLTE POLYSACCHARIDE, IN DIESEN POLYSACCHARIDEN ENTHALTENE OSE UND IHRE ANWENDUNGEN
ALTEROMONAS-TYPE BACTERIA, POLYSACCHARIDES PRODUCED BY SAID BACTERIA, OSE CONTAINED IN SAID POLYSACCHARIDES AND APPLICATIONS

(30) Priorité: 15.02.1993 FR 9301687
(43) Date de publication de la demande: 22.11.1995
(73) Titulaire: INSTITUT FRANCAIS DE RECHERCHE POUR L'EXPLOITATION DE LA MER (IFREMER), 92138 Issy-les-Moulineaux Cedex (FR)
(72) Inventeur: BARBIER, Georges, F-29200 Brest (FR); GUEZENNEC, Jean, F-29263 Plouzane (FR); PIGNET, Patricia, F-29200 Brest (FR); BOZZI, Laurent, F-38500 Voiron (FR); RINAUDO, Marguerite, F-38000 Grenoble (FR); MILAS, Michel, F-38320 Herbeys (FR); LEROY, Yves, F-59136 Wavrin (FR); DUBREUCQ, Guy, F-59111 Wavrin (FR); PRIEUR, Daniel, F-29290 Saint-Renant (FR); JEANTHON, Christian, F-29680 Roscoff (FR); VINCENT, Patrick, F-56300 Pontivy (FR); FOURNET, Bernard +di, / (FR)
(74) Mandataire: Phélip, Bruno
(86) Numéro de dépôt international: FR9400169
(87) Numéro de publication internationale: WO9418340

(56) Documents cités:
- EP-A- 0 084 333
- US-A- 4 740 466
- FOOD HYDROCOLLOIDS, vol.5, no.1/2, 1991, NEW YORK US pages 171 - 172 F.TALMONT ET AL. cité dans la demande
- KIELER MEERESFORSCHUNG, SONDERHEFT, vol.8, 1991, KIEL DE pages 188 - 192 P.VINCENT ET AL. cité dans la demande

## Description

La présente invention a pour objet des bactéries du type Alteromonas productrices de polysaccharides.

Elle est également relative à ces polysaccharides et a un ose nouveau présent dans certains de ces polysaccharides ainsi qu'à leurs applications .

Des bactéries appartenant au genre Alteromonas et productrices de polysaccharides ont déjà été étudiées.

Ainsi , les brevets japonais portant les numéros de publication JP 58 121 799 et JP 58 121 798 ont pour objet des Alteromonas appartenant à l'espèce Alteromonas vaga. Ces souches produisent des polysaccharides ayant des masses comprises entre 5.10⁴ et 15.10⁴ et constitués de 25% de glucose , 25% de mannose , 21% de galactose , 14% de rhamnose et 5% de fucose (JP 58 121 799) , ou ayant des masses comprises entre 2.10⁵ et 4.10⁵ D et constitués de 53% de rhamnose, 21 % de glucose , 7% de mannose et 6% de galactose (JP 58 121 798).

Ces documents mentionnent des utilisations comme médicaments anti-cancéreux , en particulier.

On retiendra que le polysaccharide du brevet JP 58 121 799 contient obligatoirement du fucose et ne contient pas , selon le résumé de ce document , d'acide glucuronique ni de N-acétyl-galactosamine ou de N-acétyl-glucosamine.

La colonisation de sources hydrothermales dans les profondeurs des océans par des bactéries a été mise en évidence au cours des deux dernières décades.

Il a été montré que des bactéries oxydant le soufre jouent un rôle primordial dans la chaîne alimentaire de ces sources hydrothermales . Des bactéries ayant d'autres types de métabolismes ont été néanmoins isolées.

Des souches productrices de polysaccharides ont été isolées à partir de l'épiderme de l'annélide polychète Alvinella pompejana. Ces souches sont décrites de façon sommaire et de plus aucune indication n'est donnée concernant leur accessibilité.

Des polysaccharides produits par ces souches sont décrits par leurs structures dans le compte-rendu de la première Conférence Internationale sur la Biotechnologie Marine à Tokyo (Prieur et al., 1990-Current Topics in Marine Biotechnology , pages 85-88) où l'on décrit un polysaccharide issu d'une souche dénommée 1545, dans l'article de Talmont et al. (Food Hydrocolloids , volume 5, n°1/2, pages 171-172, 1991) où l'on décrit un polysaccharide d'une masse de 1100 kD et dans le compte-rendu du Congrès de Baltimore (Vincent et al., 1991 - Production of exopolysaccharides by bacteria from deep-sea hydrothermal vents. Kieler Meeresforsch., 8 : pages 188-192 ) .

Il ressort donc de l'état de la technique connu du demandeur, que l'on connaît seulement un petit nombre de souches d'origine hydrothermale productrices de polysaccharides et qu'elles ne sont pas accessibles dans leur majorité.

En outre , les structures des polysaccharides qu'elles produisent ont été très peu décrites.

Il est pourtant très intéressant de trouver de nouveaux polysaccharides en raison de leurs applications dans de nombreux domaines par exemple comme gélifiants ou comme médicaments.

Le demandeur s'est donc attaché à rechercher d'autres polysaccharides présentant des propriétés applicables industriellement .

Il a ainsi montré de manière surprenante que des souches de bactéries du type Alteromonas , se distinguant des autres espèces , produisent des polysaccharides de structure non connue et comprenant pour certains un ose dont , à sa connaissance, la structure n'a jamais été décrite .

La présente invention a donc pour objet des bactéries du type Alteromonas se distinguant des autres espèces portées à la connaissance du demandeur, telles que A.macleodii , A.colwellii et A. vaga.

Les souches objets de la présente invention possèdent toutes les caractéristiques suivantes permettant de les rapprocher du genre Alteromonas et de les distinguer des autres espèces connues du demandeur .

### Caractéristiques morphologiques:

- bacilles Gram négatifs droits, dont la taille est comprise entre 0,5 à 0,6 µm de large et 1,3 à 1,5 µm de long ,
- souches mobiles , munies d'un flagelle polaire,
- souches non-pigmentées (absence de pigments diffusibles et non-diffusibles),
- souches capsulées.

### Caractéristiques physiologiques:

- température optimale de croissance: entre 25 et 30°C
- pH optimal de croissance : entre pH 6,5 et pH 7,5
- salinité optimale de croissance: entre 20 et 40 g/l de NaCl
   entre 430 et 770 mM de Na+
- temps de génération: entre 35 et 40 minutes

Les caractéristiques biochimiques et nutritionnelles sont mentionnées dans l'exemple 1 de la présente demande.

Les bactéries présentant notamment ces caractéristiques communes peuvent être regroupées en six groupes caractérisés par la nature des polysaccharides produits.

Le premier groupe est caractérisé par une production de polysaccharides contenant du galactose et présentant des rapports molaires par rapport à cet ose
compris entre 0,6 et 1,2 pour le rhamnose,
compris entre 1 et 2 pour le mannose,
compris entre 1,1 et 1,5 pour le glucose,
compris entre 0,4 et 0,6 pour l'acide glucuronique,
inférieur à 0,07 pour la N-acétyl galactosamine, et
inférieur à 0,04 pour la N-acétyl glucosamine.

Une bactérie de ce groupe est celle déposée sous le n°I-1283 auprès de la Collection Nationale de Cultures des Microorganismes de l'Institut Pasteur (CNCM).

Le deuxième groupe est caractérisé par des bactéries produisant des polysaccharides contenant du galactose et présentant des rapports molaires par rapport à cet ose ,
compris entre 0,2 et 1,4 pour le rhamnose,
compris entre 0,1 et 0,4 pour le mannose,
compris entre 3,3 et 10,2 pour le glucose, et
compris entre 0,06 et 0,1 pour l'acide glucuronique.

Une souche particulièrement avantageuse est celle déposée sous le n° I-1287 auprès de la CNCM.

Le troisième groupe est caractérisé en ce que les bactéries produisent des polysaccharides contenant du galactose et présentant des rapports molaires par rapport à cet ose
compris entre 0,7 et 0,9 pour le glucose,
compris entre 0,3 et 0,4 pour l'acide glucuronique, et
compris entre 0,1 et 0,3 pour l'acide galacturonique.

Un tel groupe est représenté avantageusement par la souche déposée sous le n°I-1282 auprès de la CNCM.

Un quatrième groupe est celui des bactéries produisant des polysaccharides contenant du galactose et présentant des rapports molaires par rapport à cet ose
compris entre 0,3 et 0,5 pour le rhamnose,
compris entre 0,4 et 0,6 pour le mannose
compris entre 1 et 1,8 pour le glucose
compris entre 0,4 et 0,6 pour l'acide glucuronique,
compris entre 0,1 et 0,3 pour l'acide galacturonique.

Un tel groupe est représenté avantageusement par la souche déposée sous le n°I-1284 auprès de la CNCM .

Un cinquième groupe est constitué par des bactéries produisant des polysaccharides contenant du galactose et présentant des rapports molaires par rapport à cet ose
compris entre 0,2 et 0,3 pour le rhamnose,
compris entre 0,2 et 0,4 pour le mannose
compris entre 0,5 et 1,1 pour le glucose
compris entre 0,1 et 0,3 pour l'acide glucuronique, et
compris entre 0,1 et 0,3 pour l'acide galacturonique.

Un tel groupe est avantageusement représenté par la souche de bactéries déposée sous le n°I-1285 auprès de la CNCM.

Enfin, un sixième groupe est représenté par des bactéries produisant des polysaccharides contenant du galactose et présentant des rapports molaires par rapport à cet ose
compris entre 0,9 et 1,1 pour le mannose, et ,
compris entre 0,9 et 1,1 pour le glucose.

La souche déposée sous le n°I-1286 auprès de la CNCM représente avantageusement ce sixième groupe.

Les espèces du genre Alteromonas connues du demandeur se distinguent des bactéries objet de la présente invention comme suit .

Alteromonas macleodii (Baumann et al., 1972, Taxonomy of aerobic marine eubacteria . J. Bacteriol., 110: 402-429 ; Bergey's Manual of Systematic Biology, Vol.I, 1984) se distingue des souches productrices selon l'invention par plusieurs critères:
- pas d'assimilation des substrats suivants : succinate et D-ribose.
- assimilation des substrats suivants : DL-glycérate, caprate, pyruvate, N-acétylglucosamine, DL-lactate, D-mannitol, D-xylose, n-propanol et L-sérine..

A. colwellii (Weiner, Segall et Colwell, 1985, Characterization of a marine bacterium associated with crassotrea virginica. Appl. Environ. Microbiol., 49:83-90 produit un pigment soluble brun-noir (mélanine) en phase stationnaire de croissance, lorsqu'elle est cultivée sur milieu Marine Broth,
- le temps de génération de cette souche en conditions optimales (25°C, milieu Marine Broth) est de 106 minutes, soit plus du double par rapport aux souches selon l'invention ,
- cette souche répond positivement aux tests indole et nitrate réductase et négativement au test β-galactosidase (ONPG),
- cultivée en présence d'un des substrats suivants: arbutine, tréhalose, saccharose, cellobiose et mannose, aucune acidification n'est mise en évidence.

Alteromonas vaga ( Baumann, Baumann, Mandel et Allen, 1972) ( Bergey's Manual of Systematic Bacteriology, Vol.1, 1984) se distingue des bactéries selon l'invention par de nombreux critères :
- pas de croissance à 40°C,
- pas de production d'amylase, de gélatinase et de lipase,
- pas d'assimilation des substrats suivants: saccharose, mélibiose, lactose, L-tyrosine, salicine et L-leucine,
- assimilation des substrats suivants : N-acétylglucosamine, D-mannitol, citrate, sarcosine, putrescine, D-sorbitol, DL-malate, α-céto-glutarate, m-hydroxybenzoate, p-hydroxybenzoate, pyruvate, L-arabinose et D-arabinose.

Ces distinctions d'une part entre les différents groupes de bactéries selon l'invention et d'autre part entre les bactéries selon l'invention et les autres espèces d'Alteromonas mises en évidence par des caractéristiques biochimiques, nutritionnelles, ou physiologiques se retrouvent lorsqu'on effectue des profils de ribotypage des différentes bactéries en utilisant par exemple les enzymes de restriction: Hind III, EcoRI, Xho I.

Il ressort clairement de ce qui précède que les bactéries objets de la présente invention forment un groupe , voire une espèce nouvelle d'Alteromonas , se distinguant des souches et espèces portées à la connaissance du demandeur et accessibles .

La présente invention a également pour objet les polysaccharides susceptibles d'être isolés à partir de ces bactéries.

Avantageusement , un premier groupe de polysaccharides est celui contenant du galactose et présentant des rapports molaires par rapport à cet ose compris entre 0,6 et 1,2 pour le rhamnose,
compris entre 1 et 2 pour le mannose,
compris entre 1,1 et 1,5 pour le glucose,
compris entre 0,4 et 0,6 pour l'acide glucuronique,
inférieur à 0,07 pour la N-acétyl galactosamine, et
inférieur à 0,04 pour la N-acétyl glucosamine.

Préférentiellement , de tels polysaccharides présentent une masse inférieure à 10⁶D. Ils peuvent être substitués par des groupements acétyle et/ou pyruvyle.

Un second groupe est constitué par des polysaccharides contenant du galactose et présentant des rapports molaires par rapport à cet ose ,
compris entre 0,2 et 1,4 pour le rhamnose,
compris entre 0,1 et 0,4 pour le mannose,
compris entre 3,3 et 10,2 pour le glucose, et
compris entre 0,06 et 0,1 pour l'acide glucuronique.

De tels polysaccharides peuvent être substitués par des groupements acétyle et présentent avantageusement une masse de l'ordre de 10⁶D.

Un troisième groupe est constitué de polysaccharides contenant du galactose et présentant des rapports molaires par rapport à cet ose
compris entre 0,7 et 0,9 pour le glucose,
compris entre 0,3 et 0,4 pour l'acide glucuronique, et
compris entre 0,1 et 0,3 pour l'acide galacturonique.

De tels polysaccharides peuvent de plus comprendre un acide hexuronique de structure furanique substitué sur son carbone en position 3 par un résidu lactyle . Un tel acide hexuronique peut être compris dans le polysaccharide dans un rapport molaire de 0,1 à 0,5 par rapport au galactose.

Ce troisième groupe de polysaccharides peut présenter une masse de l'ordre de 4.10⁶D et être substitué par des groupements acétyle.

Avantageusement , les liaisons entre les oses sont effectuées sur le carbone en position 4 du glucose, les carbones en positions 3, ou 4 et 6 du galactose et les carbones en positions 4 ou 3 de l'acide glucuronique. Ces polysaccharides peuvent en outre être ramifiés par l'intermédiaire d'un résidu galactose.

Dans ce troisième groupe , les polysaccharides comprennent avantageusement des répétitions d'enchaînements constitués de trois galactoses , un glucose , deux acides glucuroniques , un acide galacturonique et un acide hexuronique de structure furanique substituée sur son carbone en position 3 par un résidu lactyle .

Le quatrième groupe est composé de polysaccharides contenant du galactose et présentant des rapports molaires par rapport à cet ose
compris entre 0,2 et 0,3 pour le rhamnose,
compris entre 0,2 et 0,4 pour le mannose
compris entre 0,5 et 1,1 pour le glucose
compris entre 0,1 et 0,3 pour l'acide glucuronique, et
compris entre 0,1 et 0,3 pour l'acide galacturonique.

Un tel polysaccharide peut contenir un acide hexuronique substitué sur son carbone en position 3 par un résidu lactyle , de la même façon que pour le troisième groupe de polysaccharides . Les polysaccharides de ce groupe présentent avantageusement une masse comprise entre 5.10⁵ et 2.10⁶D .

Enfin, dans un cinquième groupe , les polysaccharides sont caractérisés en ce qu'ils produisent des polysaccharides contenant du galactose et présentant des rapports molaires par rapport à cet ose
compris entre 0,9 et 1,1 pour le mannose, et
compris entre 0,9 et 1,1 pour le glucose.

Un troisième objet de la présente demande est un hexose susceptible d'être compris dans un des polysaccharides précédemment défini.

Ainsi , un tel hexose de structure furanique substituée par un résidu lactyle présente la formule suivante : 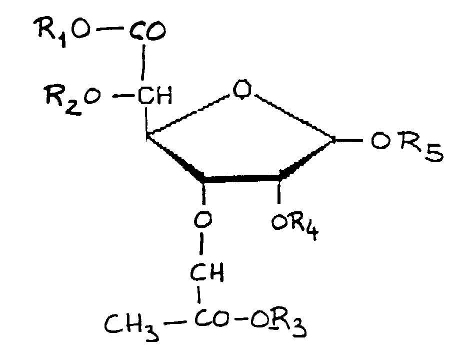 dans laquelle R¹ à R⁴ représentent chacun un atome d'hydrogène et OR₅ représente la liaison osidique avec les autres sucres.

De tels polysaccharides présentent des propriétés susceptibles de les rendre applicables industriellement , en particulier du fait de leur capacité à former des gels et émulsions, de leur pouvoir de rétention d'ions en solution et de leur activité biologique.

Ils peuvent donc être utilisés dans une large variété d'applications .

A titre d'exemple , il est possible de mettre à profit leur pouvoir de rétention d'ions pour le traitement de milieux contenant des ions métalliques, par exemple des ions calcium ou de métaux lourds (plomb, cadmium et mercure ) ou de certains radionucléides ( uranium, thorium) . Les applications concernées par un tel traitement appartiennent au domaine de la bioépuration , de la détoxification de l'environnement et de la lutte contre la pollution.

Dans de telles applications, le milieu à traiter, avantageusement sous forme de solution ou de suspension, est mis en présence avec une quantité efficace d'au moins un polysaccharide selon l'invention , qui retient les ions métalliques , après quoi on sépare de manière conventionnelle le milieu épuré et le(s) polysaccharide(s) chargé (s) des ions métalliques à éliminer .

Egalement , les nouveaux polysaccharides selon l'invention possèdent des activités biologiques , qui permettent de les utiliser comme agents thérapeutiques ou de diagnostic . En particulier , ils présentent des pouvoirs anticoagulant et/ou antithrombotique, pouvant être mis à profit dans des traitements préventifs ou curatifs d'affections liées au sang et à la circulation sanguine.

Pour ce qui est du domaine agroalimentaire , la capacité à former des gels et émulsions des nouveaux polysaccharides peut être notamment valorisée dans les applications usuelles des polysaccharides, par exemple pour la réalisation de crèmes , de glaces, de produits de charcuterie et autres.

Les présents polysaccharides peuvent être obtenus par culture des souches les produisant dans un milieu de culture adapté puis par extraction de ces polysaccharides par des méthodes connues en soi, et illustrées par exemple dans l'ouvrage de P.A. Sanford et A. Laskin : " Extracellular microbial polysaccharides ", Americ. Chem. Soc. (1977) Symposium Series n° 45.

Les polysaccharides peuvent alors être purifiés sur une colonne de chromatographie d'échange d'anions.

La présente invention est illustrée par les exemples qui suivent , dans lesquels :

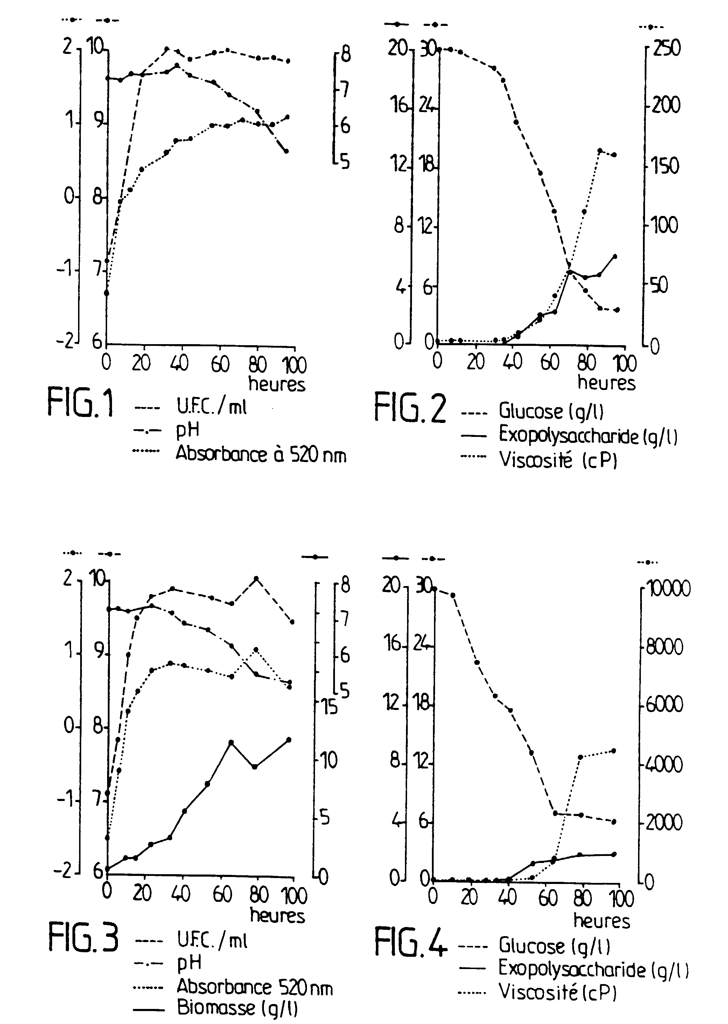

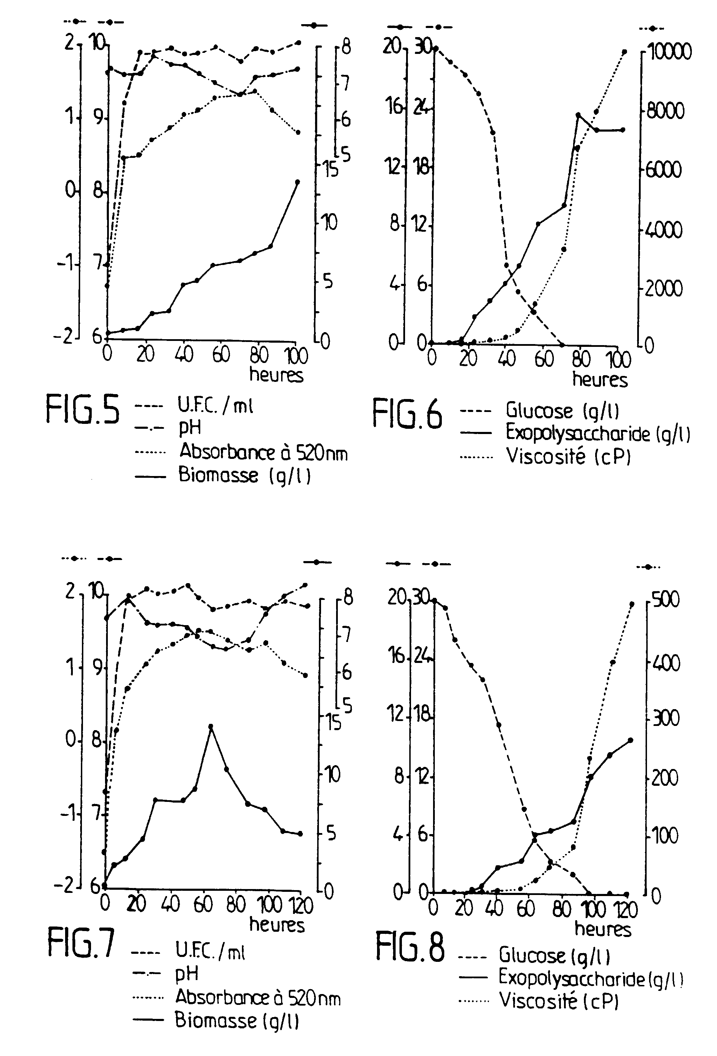

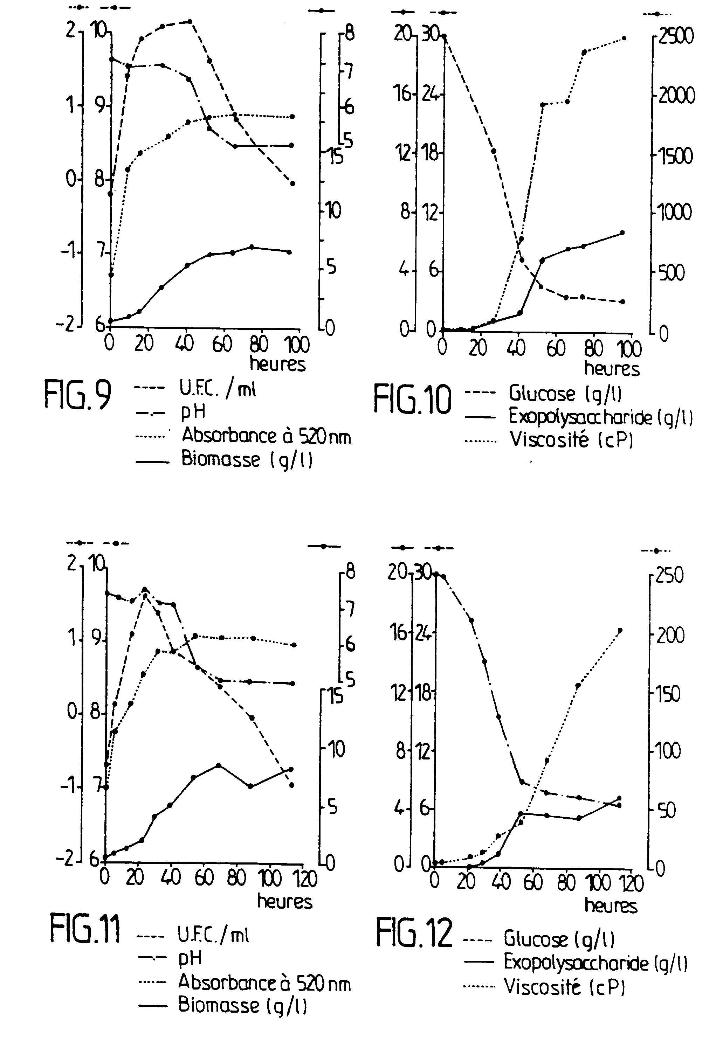

Les figures 1, 3, 5, 7, 9 et 11 illustrent la croissance , la modification du pH , l'absorbance et la biomasse ( en ordonnée) en fonction du temps de culture ( en abscisse ) pour des souches représentatives des groupes énumérés ci-dessus, à savoir respectivement les souches 342 (groupe 2 ) I-1286 ( groupe 6 ) , I-1283 (groupe 1), I-1284 ( groupe 4 ) , 1625 et I-1282 ( groupe 3).

Les figures 2, 4, 6, 8, 10 et 12 illustrent la consommation de sucre, la quantité d'exopolysaccharide produite et la viscosité du milieu de culture ( en ordonnée) en fonction du temps de culture ( en abscisse) pour des souches représentatives des groupes énumérés ci-dessus, à savoir respectivement les souches 342 ( groupe 2), I-1286 ( groupe 6 ) I-1283 (groupe 1 ) , I-1284 (groupe 4 ) , 1625 et I-1282 (groupe 3 ).

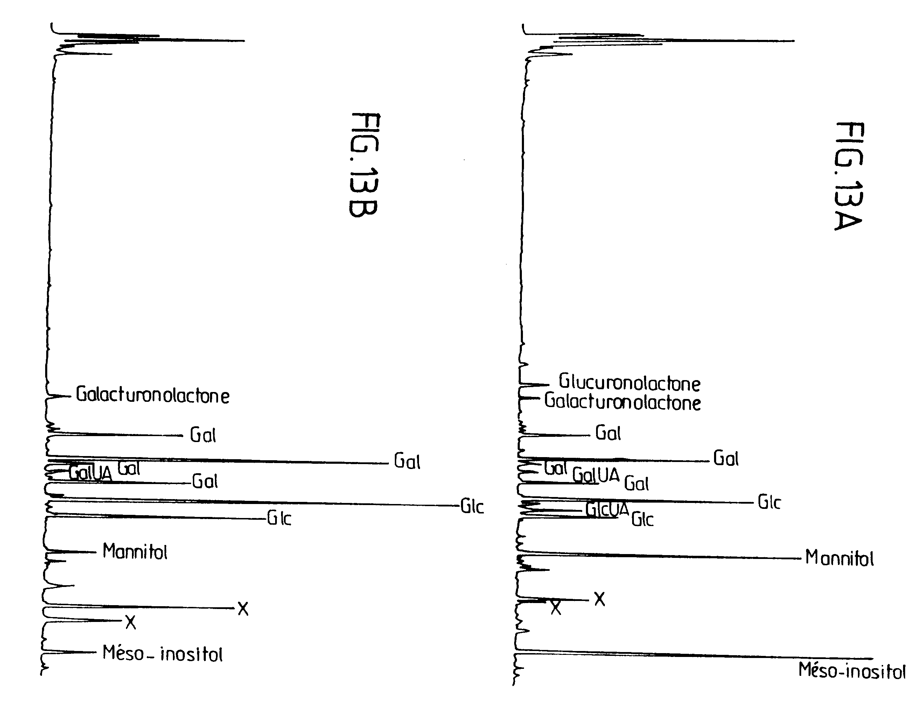

Les figures 13A et 13B représentent des chromatographes en phase gazeuse du polysaccharide de la souche I-1282 après méthanolyse et triméthylsilylation ( figure 13A) , et après réduction, méthanolyse et triméthylsilylation ( figure 13B) .

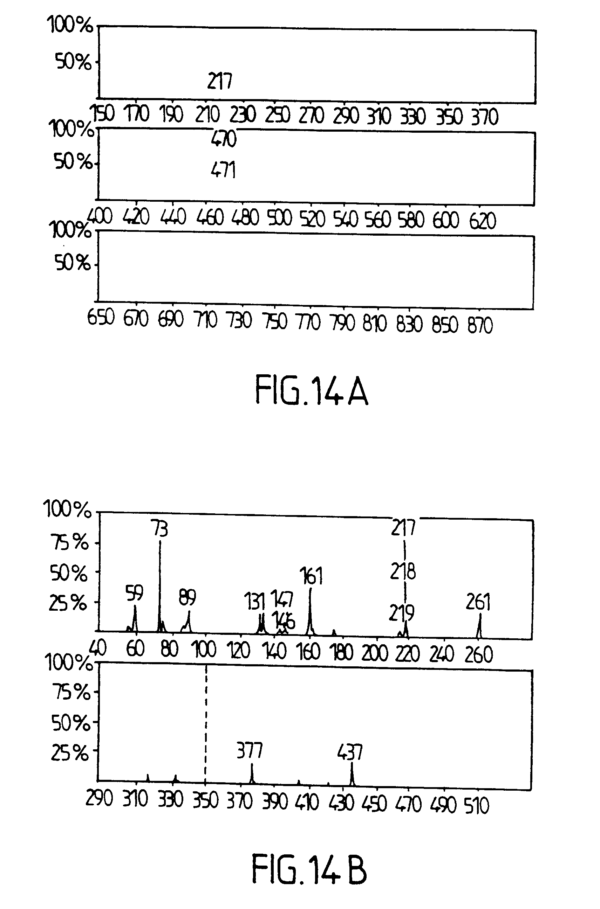

Les figures 14A et 14B représentent des spectres de masse en mode d'ionisation chimique (figure 14A) et en mode d'ionisation électronique (figure 14B) des deux pics X libérés par méthanolyse du polysaccharide purifié et analysé par chromatographie en phase gazeuse ( CPG/SM ) après triméthylsilylation.

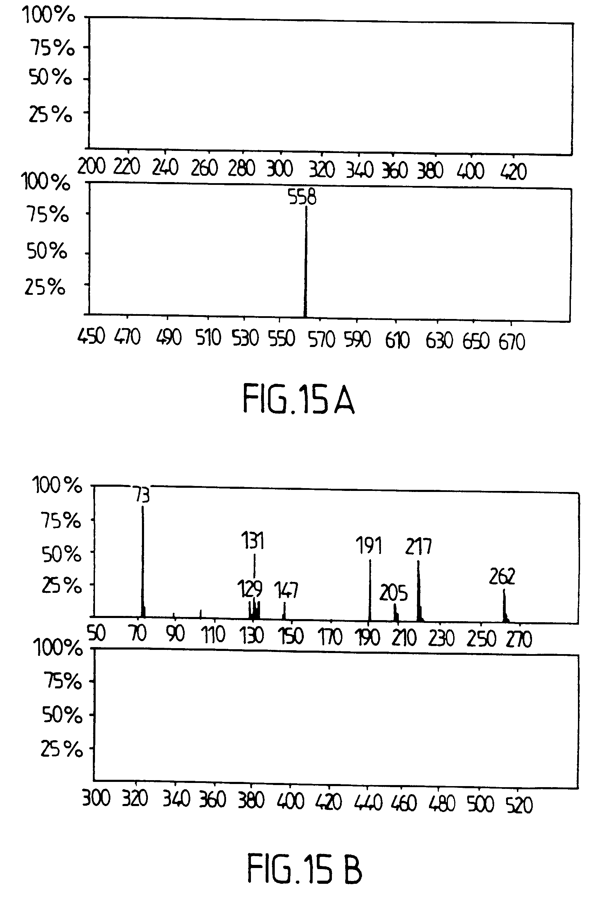

Les figures 15A et 15B représentent des spectres de masse en mode d'ionisation chimique (figure 15A) et en mode d'ionisation électronique (figure 15B) des deux pics X obtenus après méthanolyse du polysaccharide purifié réduit et analysé par CPG/SM après triméthylsilylation.

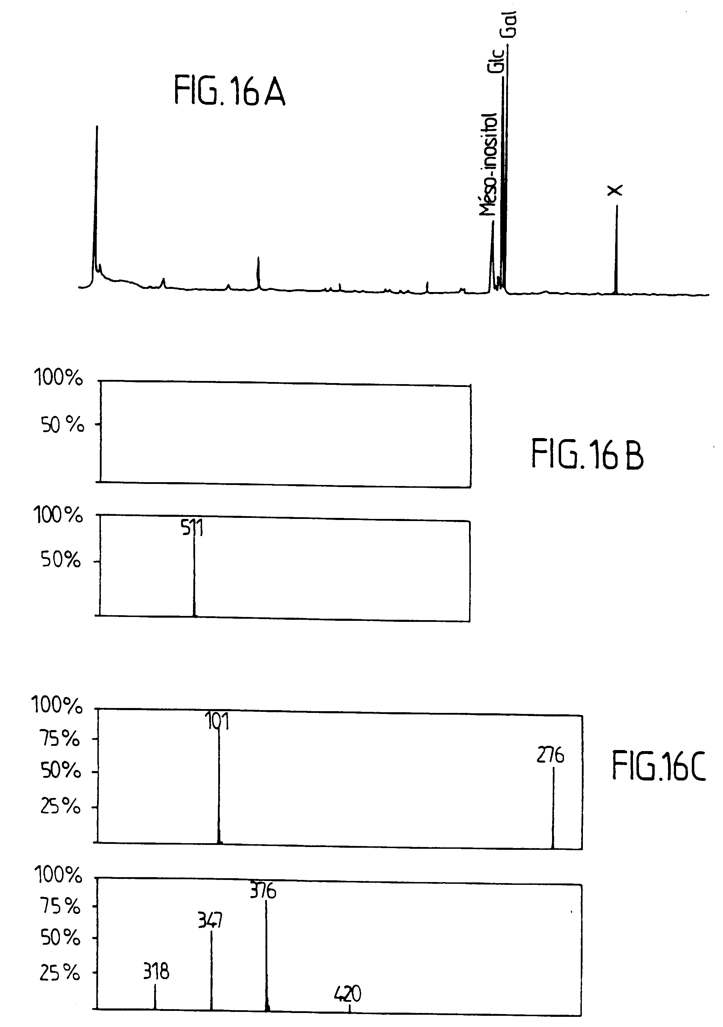

La figure 16 A est un chromatogramme en phase gazeuse des polyolacétates obtenus après réduction du polysaccharide , hydrolyse totale , réduction par BD₄K et peracétylation. Les figures 16 B et 16 C sont respectivement les spectres de masse du composé X en mode d'ionisation chimique ( figure 16 B) et en mode d'ionisation électronique ( figure 16 C) correspondant au chromatogramme de la figure 16 A.

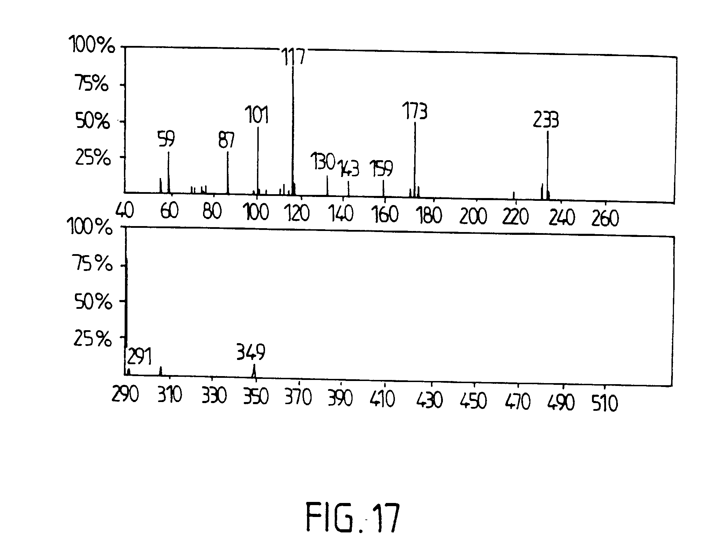

La figure 17 représente un spectre de masse en mode d'ionisation électronique de l'éther méthylique du composé X. EXEMPLE 1 :

### Caractéristiques biochimiques et réponses aux tests nutritionnels des souches selon l'invention.

### Caractéristiques biochimiques:

| Réponse positive: | Réponse négative: |
|---|---|
| - catalase | - fermentation du glucose |
| - oxydase | - production d'indole |
| - β galactosidase (ONPG) | - chitinase |
| - gélatinase | - accumulation du PHB (polyhydroxybutyrate) |
| - lipase | - citrate |
| - production d'H₂S/cystéine | - nitrate réductase |
| | - nitrite réductase |

Toutes les souches, sauf la souche I-1284 du groupe 4 , ont donné une réponse négative au test arginine dihydrolase.

Toutes les souches, sauf les souches 342 et I-1287 du groupe 2 , ont donné une réponse positive au test amylase.

### Détermination du G-C% :

Toutes les souches ont un G-C% compris entre 40 et 50.

### Réponses aux tests nutritionnels.

### I. Réponses positives communes aux souches.

Les 21 substrats assimilés communément par les souches sont présentés ci-dessous en fonction du milieu utilisé :
- milieu BM ( Baumann et Baumann, 1981- The Prokaryotes, Vol.2 : 1302-1331) ;
- milieu ASW ( Ruby et Jannasch, 1982 J. Bacteriol., 149 : 161-165 ).

### A. Assimilation sur milieu BM et milieu ASW:

| | | | |
|---|---|---|---|
| Fructose | Glucose | Maltose | Lactose |
| Cellobiose | Saccharose | Tréhalose | Galactose |
| Tyrosine | Leucine | Glycérol | Ribose |
| Succinate ( réponse faible sur milieu BM) | | | |
| Lactate ( réponse faible sur milieu BM). | | | |

Pour certains substrats, les souches n'ont été testées que sur l'un des deux milieux.

### B. Assimilation sur milieu BM:

| | | | |
|---|---|---|---|
| Lévulose | Mélibiose | Mélézitose | Raffinose |

### C. Assimilation sur milieu ASW:

| | | |
|---|---|---|
| Salicine | Propionate | Gluconate |

Dans le cas du fructose, lactose, cellobiose, tréhalose, mélibiose, mélézitoze et D-raffinose, les tests ont montré une très faible acidification pour les souches 342 et I-1287 par rapport aux autres souches . L'acidification est également très faible pour la souche I-1285 du groupe 5 dans le cas du tréhalose et pour les souches 1625 et I-1282 dans le cas du mélibiose.

Le gluconate est assimilé par toutes les souches mais aucune acidification n'a été mise en évidence . Enfin, dans le cas du glycérol, les souches 342 et I-1287 n'ont pas acidifié et l'acidification est très faible pour les autres souches .

### II. Réponses négatives communes aux souches :

### 41 substrats ne sont pas assimilés.

### A. Pas d'assimilation sur milieu BM et milieu ASW:

| | | | |
|---|---|---|---|
| D-arabinose | L-arabinose | Xylose | Sérine |
| citrate | Malate | | |

### B. Pas d'assimilation sur milieu BM:

| | | | |
|---|---|---|---|
| Rhamnose | Sorbose | Méthanol | Méthylamine |
| Pyruvate | Aspartate | Méthionine | Valine |
| Lysine | Tryptophase | Thréonine | Citruline |
| Glycérate | Glucuronate | L-ornithine | Adénine |
| 5-valérate | | | |

### C. Pas d'assimilation sur milieu ASW:

| | | | |
|---|---|---|---|
| Glutarate | Mannitol | Ethanolamine | Taurine |
| Hydroxyproline | Hydroxybenzoate | 2-céto-glutarate | Propanol-1 |
| Glycogène | Sorbitol | Benzoacétate | Dulcitol |
| Sarcosine | D-glucosamine | Putrescine | Arabitol |
| n-inositol | Xanthine | | |

### III. Réponses particulières en fonction des souches

Pour un certain nombre de substrats, les réponses varient suivant les souches.

Le mannose est assimilé par la souche I-1283 .

L'acétate et l'éthanol ne sont assimilés que par les souches du groupe IV et par les souches 342 et I-1287 .

Les souches I-1286 et 722 du groupe 6 présentent des réponses particulières pour certains substrats et notamment des réponses négatives pour les suivants : glutamate, glycine, 3 hydroxy-butyrate, proline, phénylalanine , et arginine.

Dans le cas de l'alanine, les souches 342, I-1287, et I-1285 ont présenté une réponse négative comme les souches I-1283, I-1284, 1666 , I-1282 et 1625 .

Les réponses aux tests d'assimilation permettent de mettre en évidence des métabolismes très voisins pour les souches I-1286 et 722 .

### EXEMPLE 2:

### Production d'exopolysaccharides par des souches de type Alteromonas selon l'invention:

La culture des bactéries se fait dans un fermenteur de 2 l , à pression atmosphérique normale, sur milieu de culture 2216 E ( Oppenheimer et Zobell,(1952) J. Mar. Res. 11, 10-18) contenant 750 ml d'eau de mer filtrée, 250 ml d'eau distillée, 4 g de peptone, 0,01 g de FePO₄, 1 g d'extrait de levure, 15 g de bacto-agar , additionné de glucose (30 g/l) et tamponné à pH 7,2 avec de l'acide 3-[N-Morpholino]propanesulfonique (MOPS) (50mM). Le rendement est de 4,8 g/litre.

Les résultats de croissance ( en unités formant des colonies/ml) , de changement de pH , d'absorbance à 520 nm et de biomasse ou masse cellulaire en g/l sont illustrés pour les souches 342, I-1286, I-1283, I-1284, 1625 et I-1282 respectivement sur les figures 1, 3, 5, 7, 9 et 11.

La consommation de glucose est estimée par la concentration de ce sucre dans le milieu de culture (g/l) ; la quantité d'exopolysaccharide produite (g/l) et la viscosité du milieu de culture sont illustrées pour les souches 342, I-1286, I-1283, I-1284, 1625 et I-1282 respectivement sur les figures 2, 4, 6, 8, 10 et 12.

### EXEMPLE 3 :

### Extraction des polysaccharides

Les moûts de culture sont centrifugés 2 h à 24000 g afin d'éliminer le maximum d'espèces insolubles formées en partie de débris cellulaires . Le culot est séché à l'étuve à 80°C afin d'en déterminer le poids . Le surnageant est recueilli et filtré successivement sur membranes Sartorius de porosité décroissante ( de 8µm à 0,45µm) .

A la solution récupérée, on ajoute du chlorure de sodium en poudre avantageusement à 20 g/l afin d'obtenir la forme sel de sodium du polysaccharide, mais aussi pour diminuer la qualité du solvant afin de précipiter préférentiellement les polysaccharides par rapport aux autres composés présents de masses plus faibles (protéines) lors de l'addition d'alcool éthylique. La précipitation est obtenue généralement pour un rapport éthanol (EtOH)/H₂O de 1/1 (V/V). Le précipité est alors lavé abondamment avec des mélanges EtOH/H₂O à rapports croissants en EtOH ( de 50 % V/V à 100 % V/V). Les polysaccharides sont ensuite séchés sous vide à 30°C et pesés afin de déterminer la teneur des moûts en polysaccharide.

Les rendements de production des polysaccharides par les souches selon l'invention sont les suivants:
- souche I-1283: 9 g/kg de moût en moyenne ( 3 g/kg de produits insolubles)
- souches I-1287 et 342 : 4 à 6,5 g/kg de moût ( 6 à 8 g/kg de produits insolubles ),
- souches I-1282 et 1625 : 2 à 3 g/kg de moût (8 à 18 g/kg de produits insolubles),
- souches I-1284 et 1666: 3 g/kg de moût ( 15 g/kg de produits insolubles ),
- souche I-1285 3 g/kg de moût ( 12 g/kg de produits insolubles ).

### EXEMPLE 4 :

### Détermination de la composition des polysaccharides.

### 1) Méthodes utilisées.

Les polysaccharides sont hydrolysés 1 nuit à 100°C dans H₂SO₄,2M, neutralisés au carbonate de baryum, puis évaporés à sec, repris avec 200 µl H₂O et filtrés sur membrane 3 µm . Sur une moitié on élimine les sels avec une résine mixte (Amberlite M.B.3) et on injecte en HPLC sur une colonne échangeuse d'ions (CHO 682; température 85°C; éluant H₂O distillée, débit 0,5 ( ml/min) afin d'analyser la composition en sucres neutres .

L'autre partie est injectée directement sur une colonne analytique d'acides organiques ( exclusion ionique HP x 87 H; éluant H₂SO₄ 8.10⁻³ M, débit 0,6 ml/min.).

Les polysaccharides peuvent aussi être soumis à une méthanolyse afin de les mettre sous la forme de méthylglycosides N-acétylés et O-triméthylsilylés selon la méthode de Kamerling et al. ( Biochem. J.,(1975) 151, 491-495) modifiée par Montreuil et al (1986 , Glycoproteins. In Carbohydrate analysis: a practical approach. Eds Chaplin et Kennedy, I.R.L Press, Oxford, Washington D.C., 143-204). Les espèces obtenues sont alors chromatographiées.

Cette méthode permet de déterminer les sucres neutres constitutifs de la structure ainsi que leurs rapports respectifs . Elle permet de mettre en évidence la présence d'acides uroniques , mais elle ne permet pas de préciser la nature de ces sucres.

Les colonnes de chromatographie utilisées permettent de bien mettre en évidence les substituants acétate. La présence des autres substituants et du rhamnose est obtenue à partir des spectres RMN ¹H à 80°C sur un AC300 Bruker, ce qui permet de quantifier leur dosage. Dans la solution de polysaccharide à 5 g/l dans D₂O , on ajoute comme étalon du succinate de sodium à concentration connue.

La présence des groupes acétate et carboxyliques est vérifiée par spectroscopie infrarouge. Cette méthode permet d'exclure la présence de groupements sulfate ou sulfonate sur les chaînes des polysaccharides étudiés.

La proportion des sucres et groupements acides est estimée par dosage conductimétrique sur les sels des polysaccharides purifiés. Les masses moléculaires des polysaccharides ont été mesurées par diffusion de la lumière.

On a en outre mesuré le pouvoir optique rotatoire des polysaccharides ainsi que la viscométrie de leur solution, à l'aide d'un Low Shear 30 (Contraves).

Les méthodes colorimétriques suivantes sont aussi utilisées .

### Dosages des oses neutres:

Méthode de TILLMANS J. et PHILLIPPI K.((1929) Biochem.. Z.,215, 36-60) modifiée par REMINGTON C.(1931) ( Biochem. J., 25, 1062-1071).

Modification apportée: Elimination de l'interférence due aux acides uroniques dosés par la méthode de DISCHE Z.((1947) J. Biol. Chem. 167, 189-198) (Montreuil J. et Spik G., Méthodes colorimétriques de dosage des glucides totaux - Microdosage des glucides, fascicule 1.(1963).
- Réactif :: Orcinol sulfurique
- Référence :: 1 mannose/1 galactose

### Dosages des oses acides:

Deux méthodes ont été utilisées

### 1- Méthode de DISCHE (J. Biol. Chem., (1947), 167, 189-198).

Modification apportée: Elimination de l'interférence due aux hexoses dosés par la méthode de TILLMANS et PHILLIPPI (1929) modifiée par REMINGSTON (1931) précédemment cité .
- Réactif :: Carbazol sulfurique
- Référence:: Acide glucuronique

### 2- Méthode de BLUMENKRANTZ N. et ASBOE-HANSEN G.((1973)-Anal. Biochem. 54 ,484-489).

- Réactifs :: Méta-hydroxy diphényl

Tétraborate de sodium 0,0125 M dans acide sulfurique concentré.
- Référence :: Acide glucuronique.

### Dosages des osamines:

Méthode d'ELSON L.A. MORGAN W.T.J.((1933) Biochem J. 27, 1824-1828) modifiée par BELCHER et Col.

Modification apportée : Transformation du réactif d'EHRLICH selon le procédé de BLIX. (MONTREUIL et SPIK, microdosage des glucides, fascicule 1, 1963).
- Réactifs :: Solution alcaline d'acétyl-acétone Réactif d'EHRLICH
- Référence :: glucosamine.

### Dosages des protéines:

Méthode LOWRY O.H. ROSENBROUGH N.J., et al. (1951 J. Biol; Chem., 193, 265-275).
- Réactifs :: Réactif A
Réactif B
Réactif FOLIN-CIOCALTEU 1 N
- Référence :: Albumine bovine.

### 2°) Résultats:

### a) Souches I-1283 et similaires.

### Dosages colorimétriques :

- Oses neutres (Orcinol) :: 46 à 70 %
- Oses acides (Carbazol) :: 8 à 12 %
- (Blumenkrantz):: 8 à 11 %
- Osamines (Elson-Morgan):: 0,2 à 3,7 %
- Protéines (Lowry):: < = 11%

### Rapports molaires au glactose obtenus par chromatographie en phase gazeuse:

Rhamnose : 0,6 à 1,2
Mannose : 1,0 à 2,0
Galactose : 1
Glucose : 1,1 à 1,5
Acide glucuronique : 0,4 à 0,6
Acide galacturonique : 0
N acétyl-galactosamine : 0 à 0,07
N acétyl-glucosamine : 0 à 0,04.
Substituants : en général acétate, dans un cas pas de substituant et dans deux autres acétate et pyruvate Masse d'un équivalent : 1800 g à 2200 g ( soit 1 sucre acide pour 8 à 10 sucres neutres)
Transition conformationnelle : oui, induite par la force ionique et la température.

### Viscosité:

Forte influence de la force ionique, mais stable à partir de 0,02 à 0,05 M NaCl
Très bonne stabilité en température ( viscosité en général à peine réduite d'un facteur 2 après 48 heures à 90°C, parfois accrue )
Mesure de viscosité à très faible gradient de cisaillement, 25°C, 0,1 N NaCl, pour 0,4 g/l de polymère : ⁿred = 200 à 1000 cm³/g pour des masses < 10⁶.

### b) Souches n° I-1287 et 342.

### Dosages colorimétriques :

- Oses neutres (Orcinol) :: 42 à 58 %
- Oses acides (Carbazol) :: 5 à 13 %
- (Blumenkrantz):: 5 à 11 %
- Osamines (Elson-Morgan):: 1,2 et 1,7 %
- Protéines (Lowry):: 12 à 18 %.

### Rapports molaires au galactose obtenus par chromatographie en phase gazeuse:

Rhamnose : 0,2 à 1,4
Mannose : 0,1 et 0,4
Galactose : 1
Glucose : 3,3 à 10,2
Acide glucuronique : 0,06 à 0,1
Acide galacturonique : 0
N acétyl-galactosamine : 0
N acétyl-glucosamine : O
Substituants : acétate
Masse d'un équivalent : 1700 g à 1900 g
Transition conformationnelle : absence

### Viscosité:

Assez insensible à la force ionique ( présence d'agrégats)
Stabilité en température moyenne
Mesure de viscosité à très faible gradient de cisaillement 25°C, 0,1 N NaCl, pour 0,4 g/l de polymère : ⁿ red=200 à 4000 cm3/g pour des masses voisines de 10⁶.

### c) Souches I-1282 et 1625

### Dosages colorimétriques :

- Oses neutres (Orcinol) :: 38 à 56 %
- Oses acides (Carbazol) :: 32 à 46 %
- (Blumenkrantz) :: 29 à 47 %
- Osamines (Elson-Morgan) :: 0,1 à 1,0%
- Protéines (Lowry) :: <=9%

### Rapports molaires au galactose obtenus par chromatographie en phase gazeuse:

Rhamnose : 0,1 à 0,2
Mannose : 0,1 à 0,2
Galactose : 1
Glucose : 0,7 à 0,9
Acide glucuronique : 0,3 à 0,4
Acide galacturonique : 0,2
N acétyl-galactosamine : 0
N acétyl-glucosamine : 0 .
Substituants : absence pour I-1282, acétate pour 1625
Masse d'un équivalent : 280 à 320 g
Masse : 10⁶ à 4.10⁶ pour I-1282
Transition conformationnelle : oui

### Viscosité :

Forte influence de la force ionique ( 1625 : ⁿred=5000 ml/g pour 0,01M NaCl et ⁿred=1200 ml/g pour 0,1M NaCl: I-1282: ⁿred=16000 ml/g pour 0,02M NaCl et nred=4000 ml/g pour NaCl=O,lM)
Faible stabilité en température à 90°C
Mesure de viscosité à très faible gradient de cisaillement, 25°C, 0,1N NaCl, pour 0,4 g/l de polymère : ⁿred=500 à 8000 cm³/g pour des masses voisines de 5.10⁵ à 2.10⁶.

### Gélification:

Formation d'un gel à propriétés physiques originales par interaction spécifique avec les ions multivalents, tels que Ca²⁺.

### Remarque :

Précipitation du polymère avec une solution d'éthanol à une concentration de 30% ou avec des concentrations plus faibles en fonction de la concentration en ions multivalents lors de la précipitation (Ca²⁺ par exemple) , en ce qui concerne I-1282 .

### d) Souches I-1284 et 1666.

### Dosages colorimériques :

- Oses neutres (Orcinol) :: 45 à 55%
- Oses acides (Carbazol) :: 20 à 40%
- (Blumenkrantz):: 36 %
- Osamines ( Elson-Morgan) :: 0,2 à 3 %
- Protéines (Lowry):: <=3%.

### Rapports molaires au galactose obtenus par chromatographie en phase gazeuse :

Rhamnose : 0,4
Mannose : 0,5
Galactose : 1
Glucose : 1,0 à 1,8
Acide glucuronique : 0,4 à 0,6
Acide galacturonique : 0,2
N acétyl-galactosamine : 0
N acétyl-glucosamine : 0,3.
Substituants : acétate et pyruvate pour I-1284 et pour 1666
Masse d'un équivalent : 540 à 590 g
Transition conformationnelle : non

### Viscosité :

Forte influence de la force ionique : I-1284 : ⁿred=4000 ml/g pour 0,005M NaCl et ⁿred=1500 ml/g pour 0,1M NaCl; 1666 : ⁿred=2000 ml/g pour 0,01M NaCl et ⁿred=400 ml/g pour NaCl=0.1M
Faible stabilité en température à 90°C
Mesure de viscosité à très faible gradient de cisaillement, 25°C, 0,1N NaCl, pour 0,4 g/l de polymère : ⁿred=500 à 8000 cm³/g pour des masses voisines de 5.10⁵ à 2.10⁶

### Gélification :

Formation d'un gel faible par interaction spécifique avec Ca²⁺·

### e) Souche I-1285

### Dosages colorimétriques :

- Oses neutres (Orcinol):: 47 à 55 %
- Oses acides (Carbazol):: 26 à 44%
- (Blumenkrantz):: 25 à 42%
- Osamines (Elson-Morgan):: 0,2 à 1,6%
- Protéines (Lowry):: <= 5 %

### Rapports molaires au galactose obtenus par chromatographie en phase gazeuse :

Rhamnose : 0,2 à 0,3
Mannose : 0,2 à 0,4
Galactose : 1
Glucose : 0,5 à 1,1
Acide glucuronique : 0,2
Acide galacturonique : 0,2
N acétyl-galactosamine: 0
N acétyl-glucosamine : 0

Présence d'un sucre nouveau identique à celui du polysaccharide de I-1282 ; présence de fucose selon les lots .
Substituants : absence
Masse d'un équivalent : 545 g
Transition conformationnelle : non

### Viscosité :

Très forte influence de la force ionique (ⁿred=8000 ml/g pour 0,02M NaCl et ⁿred=3500 ml/g pour 0,1M NaCl) Bonne stabilité en température à 90°C ( présence d'agrégats)
Mesure de viscosité à très faible gradient de cisaillement, 215°C, 0,1N NaCl, pour 0,4 g/l de polymère : ⁿred=500 à 8000 cm³/g pour des masses voisines de 5.10⁵ à 2.10⁶

### Gélification:

Formation d'un gel faible par interaction spécifique avec des ions multivalents, tels que Ca²⁺·

### f) Souches I-1286 et 722

### Dosages colorimétriques :

- Oses neutres :: 48 % à 50 %
- Oses acides ( Carbazol ) :: 7 % à 9 %
- ( Blumenkrantz) :: 7% à 9 %
- Osamines ( Elson-Morgan):: 1% à 3 %
- Protéines ( Lowry) :: 8% à 12 %.
Rapports molaires au galactose obtenus par chromatographie en phase gazeuse :
Glucose : 0,8 à 1,2
Mannose : 0,8 à 1,2
Pas d'acides uroniques
Présence de substituants acétate et pyruvate
Masse par équivalent : 2000 g à 3000 g
Pas de transition conformationnelle
Forte viscosité :

### EXEMPLE 5 :

### Analyse structurale du polysaccharide de la souche I-1282.

### 1°) Méthodes utilisées

### a) Méthylation

Les difficultés rencontrées pour solvater le polysaccharide dans le DMSO utilisé comme solvant de la méthylation, ont incité à effectuer préalablement une peracétylation, afin de rompre les interactions polysaccharide/polysaccharide.

5 mg de polysaccharide sont dissous dans 3 ml de formamide (Merck) et peracétylés à l'aide de 1 ml de pyridine et de 1,5 ml d'anhydride acétique sous agitation pendant 24 h à température ambiante . Le polysaccharide peracétylé est ensuite dialysé contre de l'eau distillée, puis lyophilisé. Enfin, le polysaccharide peracétylé est méthylé selon la méthode de Paz-Parente et al.( (1984) Carbohydr. Res, 141, 149-164).

Le polysaccharide peracétylé est dissous dans 0,5 ml de DMSO dans un tube en verre et placé dans un bain ultrasonique pendant 30 minutes. On ajoute 0,5 ml de base de méthylation (lithium méthylsulfinyl carbanion) à la solution de polysaccharide que l'on place à nouveau au bain ultrasonique pendant 2 heures. Après congélation, 1 ml d'iodure de méthyle (Merck) est additionné et l'alkylation se déroule pendant 2 heures au bain ultrasonique puis pendant une nuit à température ambiante.Toutes ces opérations s'effectuent sous atmosphère d'argon.

La méthylation est arrêtée par addition de 10 ml d'eau. Quelques cristaux de thiosulfate de sodium sont ajoutés pour décolorer la solution . Le polysaccharide perméthylé est extrait à 4 reprises par 0,5 ml de chloroforme . Les phases organiques sont réunies , lavées abondamment à l'eau et séchées par du sulfate de sodium anhydre. Le polysaccharide perméthylé est enfin purifié par filtration sur une colonne de Sephadex LH2O et élué par un mélange méthanol/chloroforme.

Le polysaccharide perméthylé est méthanolysé pendant 24 heures à 80°C à l'aide de méthanol/HCl 0,5 N. Après évaporation sous courant d'azote, les méthylglycosides partiellement méthylés sont acétylés par le mélange pyridine anhydride, acétique (v/v) pendant une nuit à température ambiante . L'identification des éthers méthyliques est effectuée par couplage chromatographique en phase gazeuse-spectrométrie de masse (CPG/SM) selon la méthode décrite par Fournet et al. ((1981) Anal. Biochem, 116, 489-502).

### b) Coupure réductive du polysaccharide perméthylé (Rolf et Gray, (1982) J. Am. Chem. Soc., 104, 3539-3541).

Cette technique réalise le clivage de la liaison osidique en réduisant les monosaccharides sous forme d'anhydrosucres et permet en outre l'acétylation de l'hydroxyle du monosaccharide engagé dans la liaison osidique. Une solution est préparée par addition dans un tube saturé en argon, de CaH₂ (0,2 g), CH₂Cl₂ ( 9 ml), Et₃SiH (0,8 ml) et de TMSOTf (1 ml). Le tube est fermé et placé dans un dessicateur saturé en argon, 1 jour à température ambiante . Le réactif est ensuite conservé à 4°C.

Dans un tube, sont placés 3 mg de polysaccharide perméthylé ainsi que 300 µl de la solution préparée précédemment. On laisse sous agitation 24 heures à température ambiante . 15 µl d'anhydride acétique sont ensuite ajoutés et l'on remet sous agitation 20 h à température ambiante . Toutes les réactions se déroulent en milieu anhydre.

### 2°) Résultats:

### a) Nature du nouveau sucre ( composé X).

L'analyse par couplage CPG/SM en mode d'ionisation chimique des deux pics X ( figure 13A ), obtenus après méthanolyse du polysaccharide purifié suivie d'une triméthylsilylation, permet d'identifier pour ces deux pics un même ion moléculaire [M+NH4]⁺ de 470 ( figure 14A ). Les deux pics X correspondent certainement aux deux anomères d'un même méthylglycoside triméthylsilylé de masse moléculaire 452.

Les spectres de masse des deux pics X obtenus en mode d'ionisation électronique ( figure 14B ) sont identiques et permettent d'obtenir des renseignement d'ordre structural : les pics m/z 59; 161; 218; 437 correspondent respectivement aux groupements COOCH₃, COOCH₃-CH-O-Si(CH₃)₃, au fragment C₂-C₃ et au monosaccharide ayant perdu un groupement méthyle .

Afin de vérifier la présence de groupements carboxyliques dans le composé X, celui-ci a été analysé après réduction des fonctions carboxyliques selon la méthode de Dutton et al.( (1986), Carbohydr. Res., 152, 249-259) . La figure 13B fournit le chromatogramme des méthylglycosides triméthylsilylés obtenus après méthanolyse partielle, réduction des fonctions méthylesters, méthanolyse et triméthylsilylation. On note une modification des temps de rétention du composé X indiquant la transformation des fonctions carboxyliques en fonctions alcools correspondantes . Cette modification est confirmée par l'analyse en CPG/SM en mode d'ionisation chimique, qui permet d'identifier un ion de spectromètre de masse du produit X natif ([5M+18]⁺:470) et du produit X réduit ([M+18]⁺:558) permet de mettre en évidence un incrément de masse de 88 qui correspond à la transformation de deux groupements COOCH₃ en deux groupements CH₂-O-Si(CH₃)₃. Cette analyse permet de conclure à la présence de deux fonctions carboxyliques dans le composé X. Ce résultat est confirmé par l'analyse du composé X réduit en spectromètre de masse en mode d'ionisation électronique ( figure 15B ) . On constate la disparition de l'ion 59 correspondant à la fonction COOCH₃, la disparition de l'ion 161 et son remplacement par l'ion 205, la disparition de l'ion 218 et son remplacement par l'ion 262 et la disparition de l'ion 437.

Afin de préciser la structure du composé X, celui-ci a été analysé par couplage CPG/SM après réduction du polysaccharide purifié selon Dutton et al. (1986) (précédemment cités) , hydrolyse totale à l'ATFA, réduction des fonctions carbonyles apparues et peracétylation, la réduction des fonctions carbonyles par du BD₄K permettant de marquer le carbone 1 d'une masse M+1. Les résultats sont illustrés dans la figure 16 . On constate la présence de méso-inositol ( témoin interne), de glucitol, de galactitol ainsi que du produit X dont le spectre de masse obtenu en impact électronique indique clairement la substitution du carbone 3 par un groupement de type propanol.

Les résultats des analyses du monosaccharide X sous forme de méthylglycoside méthylester triméthylsilylé ( figure 14 ) , de méthylglycoside réduit triméthylsilylé (figure 15) et de polyol acétate après réduction des fonctions carboxyliques (figure 16 ) permettent de dire qu'il s'agit d'un acide uronique de type furanique possédant un substituant de type lactique sur le carbone 3. Ce résultat est confirmé , en particulier pour la nature furanique du sucre, par l'analyse des éthers méthyliques obtenus après perméthylation du polysaccharide purifié, hydrolyse totale, réduction de la fonction carbonyle par du BH₄K, méthylestérification des fonctions acides carboxyliques à l'aide du méthanol/HCl et peracétylation ( figure 17 ). La substitution du carbone 3 par l'acide lactique est confirmée par les fragments m/z 233 et 291, et la nature furanique du monosaccharide de départ l'est par la présence du fragment 349 (O-CH₃ sur le carbone 5) et l'absence des pics 277 et 175 caractéristiques de la forme pyranique.

Le composé X est donc bien un acide hexuronique furanique possédant un groupement acide lactique . Il est à noter qu'un 4-O-[(S)-1-carboxyéthyl]-D-acide glucuronique a déjà été décrit dans la littérature comme appartenant à un exopolysaccharide de Klebsiella ( Kenne et Lindberg, (1983) Bacterial Polysaccharides; In The Polysaccharides, Eds Aspinall, 2, 287-353 ). A la connaissance du demandeur , c'est la première fois qu'un acide uronique substitué par un résidu lactique de conformation furanique est identifié.

### b) Analyse structurale du polysaccharide.

Afin d'accéder à la connaissance de la structure du polysaccharide, celui-ci a été soumis à des expériences de méthylation. Cette technique permet en effet de connaître la nature des liaisons interglycosidiques . La présence d'une proportion importante d'acides uroniques dans ce polymère a incité à utiliser, après perméthylation, plusieurs méthodes de coupure des liaisons glycosidiques : méthanolyse, hydrolyse et coupure réductive . D'autre part, on a effectué sur le polysaccharide perméthylé, une réduction des fonctions méthylesters par un borohydrure alcalin suivi d'une hydrolyse totale ou d'une méthanolyse. Dans chacun des cas, les éthers méthyliques libérés sont analysés par couplage CPG/SM. Les résultats obtenus sont consignés dans le tableau 1 ci-après .

L'analyse des éthers méthyliques, obtenus à partir du polysaccharide natif perméthylé à l'aide des trois clivages, donne des résultats comparables en ce qui concerne les hexoses neutres : rapport équimolaire entre le 2,3,6 tri-O-méthylglucose, 2,4,6 tri-O-méthylgalactose et le 2,3 di-O-méthylgalactose . La présence de cet éther diméthylique ainsi que l'identification par méthanolyse et coupure réductive du 2,3,5 tri-O-méthyl-X ( monosaccharide perméthylé en position terminale non réductrice ) indique que le polysaccharide est branché au niveau du galactose . Enfin, ces résultats indiquent la présence d'acides hexuroniques diméthylés non quantifiables compte-tenu de leur labilité en milieu acide.

Des renseignements intéressants sont obtenus par analyse des éthers méthyliques obtenus à partir du polysaccharide méthylé , réduit puis soumis à méthanolyse et à hydrolyse . En effet, on identifie deux nouveaux éthers diméthyliques: le 2,4- et le 2,3 di-O-méthylglucose provenant de la réduction du 2,4-et du 2,3 di-O-méthyl acide glucuronique. On mesure aussi une augmentation du pic de 2,3 di-O-méthylgalactose lors de l'hydrolyse qui s'explique par l'élution conjointe du 2,3 di-O-méthylgalactose et du 2,3 di-O-méthylglucose lors de la chromatographie en phase gazeuse. On comptabilise désormais 3 galactoses pour 1 glucose. Cette expérience n'a cependant pas donné de nouveaux dérivés diméthylés provenant de l'acide galacturonique. On pense que la réduction de cet acide selon Dutton et al; (1986) est difficile, hypothèse qui semble confirmée par l'analyse des méthylglycosides triméthylsilylés obtenus après méthanolyse du polysaccharide réduit ( figure 13B ) qui indique la présence résiduelle d'acide galacturonique.

Les expériences de méthylation permettent donc d'identifier dans le polysaccharide les glucosides suivants:

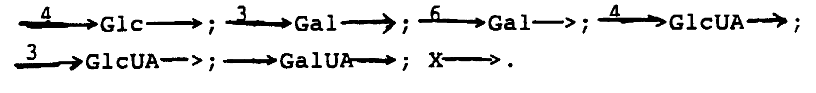

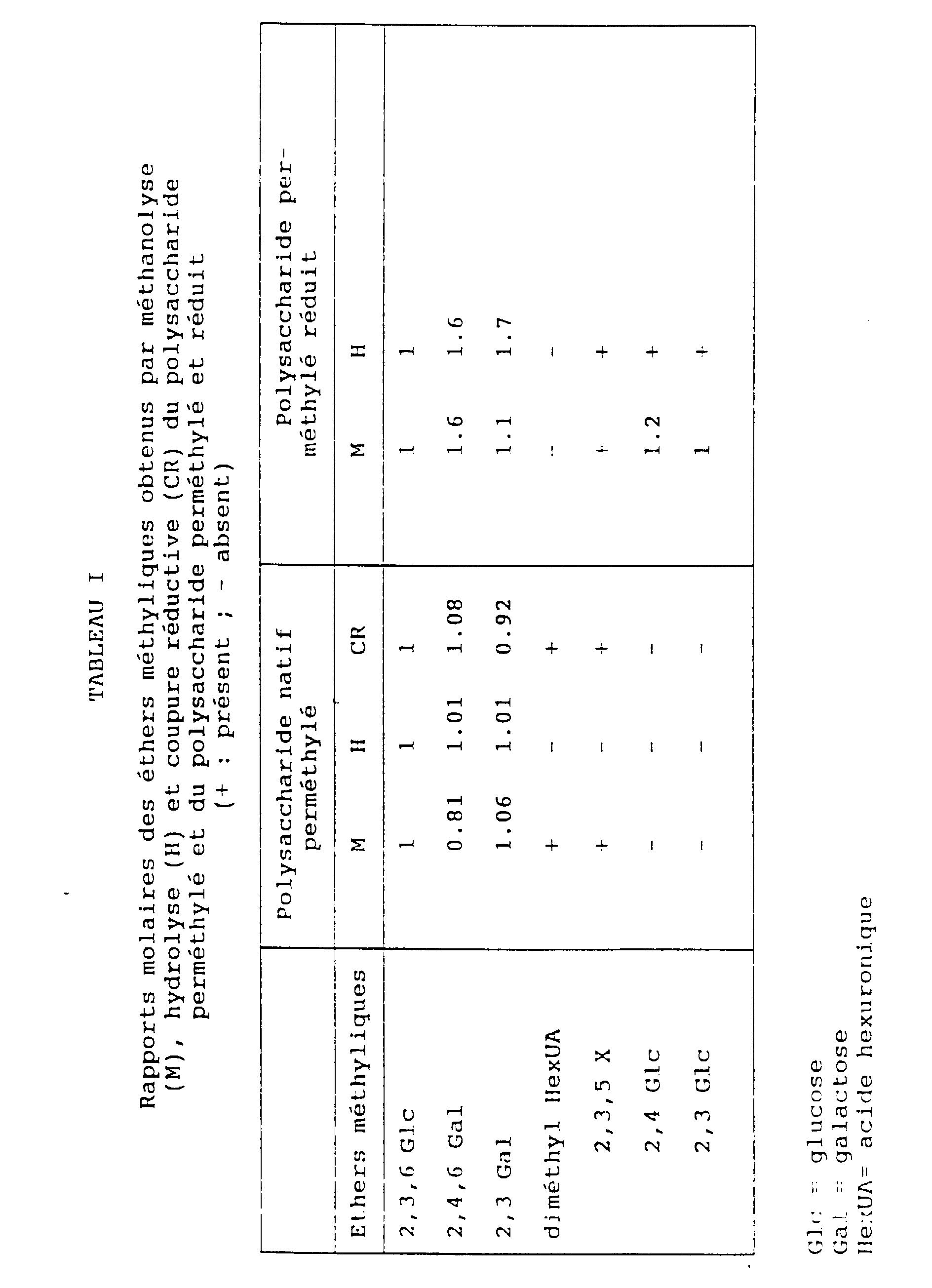

## Revendications

1. Bactéries du type Alteromonas, productrices de polysaccharides, caractérisées en ce que :
* elles se distinguent de Alteromonas macleodi en ce qu'elles:
- assimilent les substrats suivants : succinate ( sur milieux ASW et BM) et D-ribose ( sur milieux ASW et BM),
- n'assimilent pas les substrats suivants: DL-glycérate (sur milieu BM), caprate, pyruvate (sur milieu BM), N-acétylglucosamine, DL-lactate, D-mannitol ( sur milieu ASW), D-xylose (sur milieux ASW et BM), n-propanol et L-sérine ( sur milieux BM et ASW),
* elles se distinguent de A. colwellii par le fait que :
- leur temps de génération à 25°C et sur milieu Marine Broth est inférieur au moins de moitié à celui de A. colwellii,
- elles répondent négativement aux tests indole et nitrate réductase et positivement au test β-galactosidase (ONPG),
- elles produisent une acidification en culture en présence d'un des substrats suivants: arbutine, tréhalose, saccharose, cellobiose et mannose, et
* elles se distinguent de Alteromonas vaga en ce qu'elles:
- ont une croissance à 40°C,
- sont productrices d'amylase, de gélatinase et de lipase,
- assimilent les substrats suivants: saccharose ( sur milieux BM et ASW), mélibiose ( sur milieu BM), lactose ( sur milieux BM et ASW), L-tyrosine ( sur milieux BM et ASW), salicine ( sur milieu ASW) et L-leucine ( sur milieux BM et ASW),
- n'assimilent pas les substrats suivants: N-acétylglucosamine, D-mannitol ( sur milieu ASW), citrate ( sur milieux ASW et BM), sarcosine ( sur milieu ASW), putrescine ( sur milieu ASW), D-sorbitol ( sur milieu ASW), DL-malate ( sur milieux BM et ASW), α-céto-glutarate ( sur milieu ASW), m-hydroxybenzoate (sur milieu ASW), p-hydroxybenzoate (sur milieu ASW), pyruvate (sur milieu BM), L-arabinose (sur milieux ASW et BM) et D-arabinose (sur milieux ASW et BM).

2. Bactéries selon la revendication 1 caractérisées en ce qu'elles produisent des polysaccharides contenant du galactose et présentant des rapports molaires par rapport à cet ose
compris entre 0,6 et 1,2 pour le rhamnose,
compris entre 1 et 2 pour le mannose,
compris entre 1,1 et 1,5 pour le glucose,
compris entre 0,4 et 0,6 pour l'acide glucuronique,
inférieur à 0,07 pour la N-acétyl galactosamine, et
inférieur à 0,04 pour la N-acétyl glucosamine.

3. Souche de bactérie selon la revendication 2 caractérisée en ce qu'elle a été déposée sous le n° I-1283 auprès de la CNCM.

4. Bactéries selon la revendication 1 caractérisées en ce qu'elles produisent des polysaccharides contenant du galactose et présentant des rapports molaires par rapport à cet ose,
compris entre 0,2 et 1,4 pour le rhamnose,
compris entre 0,1 et 0,4 pour le mannose,
compris entre 3,3 et 10,2 pour le glucose, et
compris entre 0,06 et 0,1 pour l'acide glucuronique.

5. Souche de bactérie selon la revendication 4 caractérisée en ce qu'elle a été déposée sous le n°I-1287 auprès de la CNCM.

6. Bactéries selon la revendication 1 caractérisées en ce qu'elles produisent des polysaccharides contenant du galactose et présentant des rapports molaires par rapport à cet ose
compris entre 0,7 et 0,9 pour le glucose,
compris entre 0,3 et 0,4 pour l'acide glucuronique, et
compris entre 0,1 et 0,3 pour l'acide galacturonique.

7. Souche de bactérie selon la revendication 6 caractérisée en ce qu'elle a été déposée sous le n° I-1282 auprès de la CNCM.

8. Bactéries selon la revendication 1 caractérisées en ce qu'elles produisent des polysaccharides contenant du galactose et présentant des rapports molaires par rapport à cet ose
compris entre 0,3 et 0,5 pour le rhamnose,
compris entre 0,4 et 0,6 pour le mannose,
compris entre 1 et 1,8 pour le glucose
compris entre 0,4 et 0,6 pour l'acide glucuronique,
compris entre 0,1 et 0,3 pour l'acide galacturonique.

9. Souche de bactérie selon la revendication 8 caractérisée en ce qu'elle a été déposée sous le n° I-1284 auprès de la CNCM.

10. Bactéries selon la revendication 1 caractérisées en ce qu'elles produisent des polysaccharides contenant du galactose et présentant des rapports molaires par rapport à cet ose
compris entre 0,2 et 0,3 pour le rhamnose,
compris entre 0,2 et 0,4 pour le mannose
compris entre 0,5 et 1,1 pour le glucose
compris entre 0,1 et 0,3 pour l'acide glucuronique, et
compris entre 0,1 et 0,3 pour l'acide galacturonique.

11. Souche de bactérie selon la revendication 10 caractérisée en ce qu'elle a été déposée sous le n°I-1285 auprès de la CNCM.

12. Bactéries selon la revendication 1 caractérisées en ce qu'elles produisent des polysaccharides contenant du galactose et présentant des rapports molaires par rapport à cet ose
compris entre 0,9 et 1,1 pour le mannose, et
compris entre 0,9 et 1,1 pour le glucose.

13. Souche de bactérie selon la revendication 12 caractérisée en ce qu'elle a été déposée sous le n° I-1286 auprès de la CNCM.

14. Polysaccharide susceptible d'être isolé à partir d'une bactérie ou d'une souche de bactérie selon l'une quelconque des revendications 1 à 13 caractérisé en ce qu'il contient du galactose et présente des rapports molaires par rapport à cet ose
compris entre 0,6 et 1,2 pour le rhamnose,
compris entre 1 et 2 pour le mannose,
compris entre 1,1 et 1,5 pour le glucose, et
compris entre 0,4 et 0,6 pour l'acide glucuronique,
inférieur à 0,07 pour la N-acétyl galactosamine, et
inférieur à 0,04 pour la N-acétyl glucosamine.

15. Polysaccharide selon la revendication 14, caractérisé en ce qu'il est substitué par des groupements acétyle et/ou pyruvyle.

16. Polysaccharide selon l'une des revendications 14 et 15, caractérisé en ce qu'il présente une masse inférieure à 10⁶D.

17. Polysaccharide susceptible d'être isolé à partir d'une bactérie ou d'une souche de bactérie selon l'une quelconque des revendications 1 à 13 caractérisé en ce qu'il contient du galactose et présente des rapports. molaires par rapport à cet ose,
compris entre 0,2 et 1,4 pour le rhamnose,
compris entre 0,1 et 0,4 pour le mannose,
compris entre 3,3 et 10,2 pour le glucose, et
compris entre 0,06 et 0,1 pour l'acide glucuronique.

18. Polysaccharide selon la revendication 17 caractérisé en ce qu'il est substitué par des groupements acétyle.

19. Polysaccharide selon l'une des revendications 17 et 18, caractérisé en ce qu'il présente une masse de l'ordre de 10⁶D.

20. Polysaccharide susceptible d'être isolé à partir d'une bactérie ou d'une souche de bactérie selon l'une quelconque des revendications 1 à 13 caractérisé en ce qu'il contient du galactose et présente des rapports molaires par rapport à cet ose
compris entre 0,7 et 0,9 pour le glucose,
compris entre 0,3 et 0,4 pour l'acide glucuronique, et
compris entre 0,1 et 0,3 pour l'acide galacturonique.

21. Polysaccharide selon la revendication 20, caractérisé en ce qu'il comprend un acide hexuronique de structure furanique substitué sur son carbone en position 3 par un résidu lactyle. 21

22. Polysaccharide selon la revendication 21, caractérisé en ce que le rapport molaire de l'acide hexuronique au galactose est compris entre 0,1 et 0,5.

23. Polysaccharide selon l'une des revendications 21 et 22 caractérisé en ce qu'il présente une masse de l'ordre de 4.10⁶D.

24. Polysaccharide selon l'une des revendications 21 à 23 caractérisé en ce que les liaisons entre les oses sont réalisées sur le carbone en position 4 du glucose, les carbones en positions 3, ou 4 et 6 du galactose et les carbones en positions 4 ou 3 de l'acide glucuronique.

25. Polysaccharide selon l'une des revendications 21 à 24 caractérisé en ce qu'il est ramifié par l'intermédiaire d'un résidu galactose présentant des liaisons en positions 4 et 6.

26. Polysaccharide selon l'une des revendications 21 à 25 caractérisé en ce qu'il comprend des répétitions d'enchaînements constituées de 3 galactoses, 1 glucose, 2 acides glucuroniques, 1 acide galacturonique et 1 acide hexyronique de structure furanique, substitué sur son carbone en position 3 par un résidu lactyle.

27. Polysaccharide selon l'une des revendications 20 à 26 caractérisé en ce qu'il est substitué par des groupements acétyle.

28. Polysaccharide susceptible d'être isolé à partir d'une bactérie ou d'une souche de bactérie selon l'une quelconque des revendications 1 à 13, caractérisé en ce qu'il contient du galactose et présente des rapports molaires par rapport à cet ose
compris entre 0,2 et 0,3 pour le rhamnose,
compris entre 0,2 et 0,4 pour le mannose
compris entre 0,5 et 1,1 pour le glucose
compris entre 0,1 et 0,3 pour l'acide glucuronique, et
compris entre 0,1 et 0,3 pour l'acide galacturonique.

29. Polysaccharide selon la revendication 28 caractérisé en ce qu'il contient un acide hexuronique substitué sur son carbone en position 3 par un résidu lactyle.

30. Polysaccharide selon l'une des revendications 28 et 29 caractérisé en ce qu'il présente une masse comprise entre 5.10⁵ et 2.10⁶D.

31. Polysaccharide susceptible d'être isolé à partir d'une bactérie ou d'une souche de bactérie selon l'une quelconque des revendications 1 à 13, caractérisé en ce qu'il contient du galactose et présente des rapports molaires par rapport à cet ose
compris entre 0,9 et 1,1 pour le mannose, et
compris entre 0,9 et 1,1 pour le glucose.

32. Hexose présentant la formule suivante: 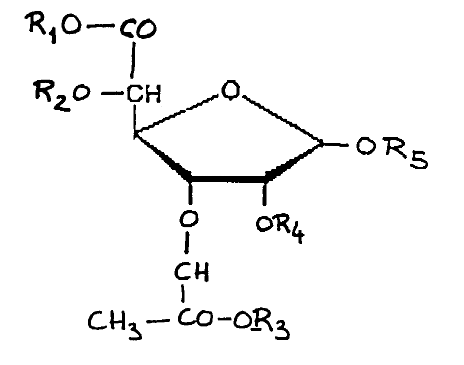 dans laquelle R₁ à R₄ représentent chacun un atome d'hydrogène et OR₅ représente la liaison osidique avec les autres sucres.

33. Hexose selon la revendication 32 caractérisé en ce qu'il est susceptible d'être compris dans le polysaccharide selon l'une des revendications 21 et 29.

34. Application de polysaccharides selon l'une quelconque des revendications 14 à 31 pour le traitement de milieux contenant des ions métalliques, par exemple des ions calcium ou de métaux lourds (plomb, cadmium et mercure) ou de certains radionucléides (uranium, thorium), le milieu à traiter, avantageusement sous forme de solution de suspension, étant mis en présence avec une quantité efficace d'au moins un desdits polysaccharides après quoi on sépare de manière conventionnelle le milieu épuré et le(s) polysaccharide(s) chargé(s) des ions métalliques à éliminer.

35. Utilisation de polysaccharides selon l'une quelconque des revendications 14 à 31, à titre d'agents de diagnostic, en particulier en raison de leurs pouvoirs anticoagulant et/ou antithrombotique.

36. Médicament contenant un polysaccharide selon l'une des revendications 14 à 31.

37. Utilisation d'un polysaccharide selon l'une des revendications 14 à 31 pour la fabrication d'un médicament pour le traitement préventif ou curatif d'affections liées au sang et à la circulation sanguine.

38. Procédé de production de polysaccharides selon l'une des revendications 14 à 31 caractérisé en ce que l'une des bactéries selon l'une des revendications 1 à 13 est mise en culture.

## Patentansprüche

1. Bakterien der Gattung Alteromonas, die Polysaccharide produzieren, dadurch gekennzeichnet, daß sie:
* sich von Alteromonas macleodi dadurch unterscheiden, daß sie:
- die folgenden Substrate assimilieren: Succinat (im ASW- und BM-Milieu) und D-Ribose (im ASW-und BM-Milieu),
- die folgenden Substrate nicht assimilieren: DL-Glycerat (im BM-Milieu), Caprat, Pyruvat, (im BM-Milieu), N-Acetylglucosamin, DL-Lactat, D-Mannit (im ASW-Milieu), D-Xylose (im ASW- und BM-Milieu), n-Propanol und L-Serin (im BM- und ASW-Milieu),
* sich von A. colwellii dadurch unterscheiden, daß:
- ihre Verdoppelungszeit bei 25 °C und im Marine Broth-Milieu um mindestens die Hälfte niedriger als diejenige von A. colwellii ist,
- sie negativ auf den Indol- und Nitratreduktase-Test und positiv auf den β-Galactosidase-Test (ONPG) reagieren,
- sie in Kultur eine Ansäuerung in Gegenwart eines der folgenden Substrate erzeugen: Arbutin, Trehalose, Saccharose, Cellobiose und Mannose, und
* sich von Alteromonas vaga dadurch unterscheiden, daß sie:
- ein Wachstum bei 40 °C aufweisen,
- Amylase, Gelatinase und Lipase produzieren,
- die folgenden Produkte assimilieren: Saccharose (im BM- und ASW-Milieu), Melibiose (im BM-Milieu), Lactose (im BM- und ASW-Milieu), L-Tyrosin (im BM- und ASW-Milieu), Salicin (im ASW-Milieu) und L-Leucin (im BM- und ASW-Milieu),
- die folgenden Substrate nicht assimilieren: N-Acetylglucosamin, D-Mannit (im ASW-Milieu), Citrat (im ASW- und BM-Milieu), Sarcosin (im ASW-Milieu), Putrescin (im ASW-Milieu), D-Sorbit (im ASW-Milieu), DL-Malat (im BM- und ASW-Milieu), α-Ketoglutarat (im ASW-Milieu), m-Hydroxybenzoat (im ASW-Milieu), p-Hydroxybenzoat (im ASW-Milieu), Pyruvat (im BM-Milieu), L-Arabinose (im ASW- und BM-Milieu), und D-Arabinose (im ASW- und BM-Milieu).

2. Bakterien nach Anspruch 1, dadurch gekennzeichnet, daß sie Polysaccharide erzeugen, die Galactose enthalten und in bezug auf dieses Monosaccharid Stoffmengen-Verhältnisse aufweisen, die
zwischen 0,6 und 1,2 für Rhamnose,
zwischen 1 und 2 für Mannose,
zwischen 1,1 und 1,5 für Glucose,
zwischen 0,4 und 0,6 für Glucuronsäure liegen,
weniger als 0,07 für N-Acetylgalactosamin und
weniger als 0,04 für N-Acetylglucosamin betragen.

3. Bakterienstamm nach Anspruch 2, dadurch gekennzeichnet, daß er unter der Nr. I-1283 beim CNCM hinterlegt worden ist.

4. Bakterien nach Anspruch 1, dadurch gekennzeichnet, daß sie Polysaccharide erzeugen, die Galactose enthalten und in bezug auf dieses Monosaccharid Stoffmengen-Verhältnisse aufweisen, die
zwischen 0,2 und 1,4 für Rhamnose,
zwischen 0,1 und 0,4 für Mannose,
zwischen 3,3 und 10,2 für Glucose und
zwischen 0,06 und 0,1 für Glucuronsäure liegen.

5. Bakterienstamm nach Anspruch 4, dadurch gekennzeichnet, daß er unter der Nr. I-1287 beim CNCM hinterlegt worden ist.

6. Bakterien nach Anspruch 1, dadurch gekennzeichnet, daß sie Polysaccharide erzeugen, die Galactose enthalten und in bezug auf dieses Monosaccharid Stoffmengen-Verhältnisse aufweisen, die
zwischen 0,7 und 0,9 für Glucose,
zwischen 0,3 und 0,4 für Glucuronsäure und
zwischen 0,1 und 0,3 für Galacturonsäure liegen.

7. Bakterienstamm nach Anspruch 6, dadurch gekennzeichnet, daß er unter der Nr. I-1282 beim CNCM hinterlegt worden ist.

8. Bakterien nach Anspruch 1, dadurch gekennzeichnet, daß sie Polysaccharide erzeugen, die Galactose enthalten und in bezug auf dieses Monosaccharid Stoffmengen-Verhältnisse aufweisen, die
zwischen 0,3 und 0,5 für Rhamnose,
zwischen 0,4 und 0,6 für Mannose,
zwischen 1 und 1,8 für Glucose,
zwischen 0,4 und 0,6 für Glucuronsäure,
zwischen 0,1 und 0,3 für Galacturonsäure liegen.

9. Bakterienstamm nach Anspruch 8, dadurch gekennzeichnet, daß er unter der Nr. I-1284 beim CNCM hinterlegt worden ist.

10. Bakterien nach Anspruch 1, dadurch gekennzeichnet, daß sie Polysaccharide erzeugen, die Galactose enthalten und in bezug auf dieses Monosaccharid Stoffmengen-Verhältnisse aufweisen, die
zwischen 0,2 und 0,3 für Rhamnose,
zwischen 0,2 und 0,4 für Mannose,
zwischen 0,5 und 1,1 für Glucose,
zwischen 0,1 und 0,3 für Glucuronsäure und
zwischen 0,1 und 0,3 für Galacturonsäure liegen.

11. Bakterienstamm nach Anspruch 10, dadurch gekennzeichnet, daß er unter der Nr. I-1285 beim CNCM hinterlegt worden ist.

12. Bakterien nach Anspruch 1, dadurch gekennzeichnet, daß sie Polysaccharide erzeugen, die Galactose enthalten und in bezug auf dieses Monosaccharid Stoffmengen-Verhältnisse aufweisen, die
zwischen 0,9 und 1,1 für Mannose und
zwischen 0,9 und 1,1 für Glucose betraaen.

13. Bakterienstamm nach Anspruch 12, dadurch gekennzeichnet, daß er unter der Nr. I-1286 beim CNCM hinterlegt worden ist.

14. Polysaccharid, das aus einer Bakterie oder einem Bakterienstamm nach einem der Ansprüche 1 bis 13 isolierbar ist, dadurch gekennzeichnet, daß es Galactose enthält und in bezug auf dieses Monosaccharid Stoffmengen-Verhältnisse aufweist, die
zwischen 0,6 und 1,2 für Rhamnose,
zwischen 1 und 2 für Mannose,
zwischen 1,1 und 1,5 für Glucose und
zwischen 0,4 und 0,6 für Glucuronsäure liegen,
weniger als 0,07 für N-Acetylgalactosamin und
weniger als 0,04 für N-Acetylglucosamin betragen.

15. Polysaccharid nach Anspruch 14, dadurch gekennzeichnet, daß es durch Acetyl- und/oder Pyruvylgruppen substituiert ist.

16. Polysaccharid nach einem der Ansprüche 14 und 15, dadurch gekennzeichnet, daß es eine Masse von weniger als 106 D aufweist.

17. Polysaccharid, das aus einer Bakterie oder einem Bakterienstamm nach einem der Ansprüche 1 bis 13 isolierbar ist, dadurch gekennzeichnet, daß es Galactose enthält und in bezug auf dieses Monosaccharid Stoffmengen-Verhältnisse aufweist, die
zwischen 0,2 und 1,4 für Rhamnose,
zwischen 0,1 und 0,4 für Mannose,
zwischen 3,3 und 10,2 für Glucose und
zwischen 0,06 und 0,1 für Glucuronsäure liegen.

18. Polysaccharid nach Anspruch 17, dadurch gekennzeichnet, daß es durch Acetylgruppen substituiert ist.

19. Polysaccharid nach einem der Ansprüche 17 und 18, dadurch gekennzeichnet, daß es eine Masse in der Größenordnung von 10⁶ D aufweist.

20. Polysaccharid, das aus einer Bakterie oder einem Bakterienstamm nach einem der Ansprüche 1 bis 13 isolierbar ist, dadurch gekennzeichnet, daß es Galactose enthält und in bezug auf dieses Monosaccharid Stoffmengen-Verhältnisse aufweist, die
zwischen 0,7 bis 0,9 für Glucose,
zwischen 0,3 und 0,4 für Glucuronsäure und
zwischen 0,1 und 0,3 für Galacturonsäure liegen.

21. Polysaccharid nach Anspruch 20, dadurch gekennzeichnet, daß es eine Hexuronsäure mit einer Furanstruktur enthält, die an deren Kohlenstoff in Position 3 durch einen Lactylrest substituiert ist.

22. Polysaccharid nach Anspruch 21, dadurch gekennzeichnet, daß das Stoffmengen-Verhältnis von Hexuronsäure zu Galactose zwischen 0,1 und 0,5 liegt.

23. Polysaccharid nach einem der Ansprüche 21 und 22, dadurch gekennzeichnet, daß es eine Masse in der Größenordnung von 4·10⁶D aufweist.

24. Polysaccharid nach einem der Ansprüche 21 bis 23, dadurch gekennzeichnet, daß die Bindungen zwischen den Monosacchariden über den Kohlenstoff in Position 4 der Glucose, die Kohlenstoffe in den Positionen 3 oder 4 und 6 der Galactose und die Kohlenstoffe in den Positionen 4 oder 3 der Glucuronsäure bestehen.

25. Polysaccharid nach einem der Ansprüche 21 bis 24, dadurch gekennzeichnet, daß es durch das Zwischenglied eines Galactoserests verzweigt ist, der Bindungen in den Positionen 4 und 6 aufweist.

26. Polysaccharid nach einem der Ansprüche 21 bis 25, dadurch gekennzeichnet, daß es Repetiereinheiten umfaßt, bestehend aus drei Galactosen, einer Glucose, zwei Glucuronsäuren, einer Galacturonsäure und einer Hexuronsäure mit einer Furanstruktur, die an deren Kohlenstoff in Position 3 durch einen Lactylrest substituiert ist.

27. Polysaccharid nach einem der Ansprüche 20 bis 26, dadurch gekennzeichnet, daß es durch Acetylgruppen substituiert ist.

28. Polysaccharid, das aus einer Bakterie oder einem Bakterienstamm nach einem der Ansprüche 1 bis 13 isolierbar ist, dadurch gekennzeichnet, daß es Galactose enthält und in bezug auf dieses Monosaccharid Stoffmengen-Verhältnisse aufweist, die
zwischen 0,2 und 0,3 für Rhamnose,
zwischen 0,2 und 0,4 für Mannose,
zwischen 0,5 bis 1,1 für Glucose,
zwischen 0,1 und 0,3 für Glucuronsäure und
zwischen 0,1 und 0,3 für Galacturonsäure liegen.

29. Polysaccharid nach Anspruch 28, dadurch gekennzeichnet, daß es eine Hexuronsäure enthält, die an ihrem Kohlenstoff in Position 3 durch einen Lactylrest substituiert ist.

30. Polysaccharid nach einem der Ansprüche 28 und 29, dadurch gekennzeichnet, daß es eine Masse zwischen 5·10⁵ und 2·10⁶ D aufweist.

31. Polysaccharid, das aus einer Bakterie oder einem Bakterienstamm nach einem der Ansprüche 1 bis 13 isolierbar ist, dadurch gekennzeichnet, daß es Galactose enthält und in bezug auf dieses Monosaccharid Stoffmengen-Verhältnisse aufweist, die
zwischen 0,9 und 1,1 für Mannose und
zwischen 0,9 und 1,1 für Glucose liegen.

32. Hexose, die die folgende Formel aufweist: 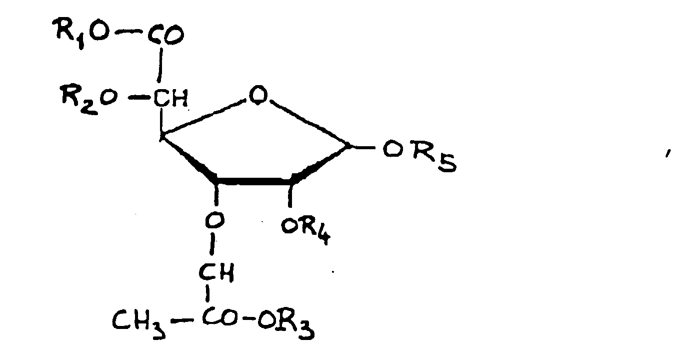 worin R₁ bis R₄ jeweils ein Wasserstoffatom darstellen und OR₅ eine Sauerstoffbindung mit den anderen Zuckern darstellt.

33. Hexose nach Anspruch 32, dadurch gekennzeichnet, daß sie in dem Polysaccharid nach einem der Ansprüche 21 und 29 enthalten sein kann.

34. Anwendung von Polysacchariden nach einem der Ansprüche 14 bis 31 zur Behandlung von Milieus, die Metallionen, beispielsweise Calciumionen oder Ionen von Schwermetallen (Blei, Cadmium und Quecksilber) oder bestimmten Radionukliden (Uran, Thorium) enthalten, wobei das zu behandelnde Milieu, vorteilhaft in Form einer Lösungs-Suspension, mit einer wirksamen Menge mindestens eines der Polysaccharide zusammengebracht wird, wonach man das gereinigte Milieu und das (die) mit den zu eliminierenden Metallionen beladene(n) Polysaccharid(e) auf herkömmliche Weise trennt.

35. Verwendung von Polysacchariden nach einem der Ansprüche 14 bis 31 als Diagnostika, insbesondere aufgrund ihres antikoagulierenden und antithrombotischen Vermögens.

36. Medikament, das ein Polysaccharid nach einem der Ansprüche 14 bis 31 enthält.

37. Verwendung eines Polysaccharids nach einem der Ansprüche 14 bis 31 zur Herstellung eines Medikaments zur vorbeugenden oder heilenden Behandlung von Erkrankungen im Zusammenhang mit dem Blut und dem Blutkreislauf.

38. Verfahren zur Herstellung von Polysacchariden nach einem der Ansprüche 14 bis 31, dadurch gekennzeichnet, daß eine der Bakterien nach einem der Ansprüche 1 bis 13 kultiviert wird.

## Claims

1. Polysaccharide-producing bacteria of the Alteromonas type, characterized in that :
* they are distinguished from Alteromonas macleodii in that they :
- assimilate the following substrates : succinate (on ASW and BM media) and D-ribose (on ASW and BM media),
- do not assimilate the following substrates : DL-glycerate (on BM medium). caprate, pyruvate (on BM medium), N-acetylglucosamine, DL-lactate, D-mannitol (on ASW medium), D-xylose (on ASW and BM media), n-propanol and L-serine (on BM and ASW media),
* they are distinguished from A. colwellii by the fact that :
- their generation time at 25°C and on Marine Broth medium is less than half of that of A. colwellii,
- they respond negatively to the indole and nitrate reductase tests and positively to the β-galactosidase test (ONPG),
- they produce an acidification in culture in the presence of one of the following substrates : arbutine, trehalose, saccharose, cellobiose and mannose, and
* they are distinguished from Alteromonas vaga in that they :
- grow at 40°C,
- produce amylase, gelatinase and lipase,
- assimilate the following substrates : saccharose (on BM and ASW media). melibiose (on BM medium), lactose (on BM and ASW media), L-tyrosine (on BM and ASW media), salicine (on ASW medium) and L-leucine (on BM and ASW media),
- do not assimilate the following substrates : N-acetylglucosamine, D-mannitol (on ASW medium), citrate (on ASW and BM media), sarcosine (on ASW medium), putrescine (on ASW medium), D-sorbitol (on ASW medium), DL-malate (on BM and ASW media), α-keto-glutarate (on ASW medium), m-hydroxybenzoate(on ASW medium), p-hydroxybenzoate (on ASW medium), pyruvate (on BM medium), L-arabinose (on ASW and BM media) and D-arabinose (on ASW and BM media).

2. Bacteria according to claim 1, characterized in that they produce polysaccharides containing galactose and having molar ratios with respect to this ose of between 0.6 and 1.2 for rhamnose, between 1 and 2 for mannose, between 1.1 and 1.5 for glucose, between 0.4 and 0.6 for glucuronic acid, less than 0.07 for N-acetyl galactosamine, and less than 0.04 for N-acetyl glucosamine.

3. Strain of bacteria according to claim 2, characterized in that it has been deposited at the CNCM under the n° I-1283.

4. Bacteria according to claim 1, characterized in that they produce polysaccharides containing galactose and having molar ratios with respect to this ose of between 0.2 and 1.4 for rhamnose, between 0.1 and 0.4 for mannose, between 3.3 and 10.2 for glucose, and between 0.06 and 0.1 for glucuronic acid.

5. Strain of bacteria according to claim 4, characterized in that it has been deposited at the CNCM under the n° I-1287.

6. Bacteria according to claim 1, characterized in that they produce polysaccharides containing galactose and having molar ratios with respect to this ose of between 0.7 and 0.9 for glucose, between 0.3 and 0.4 for glucuronic acid, and between 0. 1 and 0.3 for galacturonic acid.

7. Strain of bacteria according to claim 6, characterized in that it has been deposited at the CNCM under the n° I-1282.

8. Bacteria according to claim 1, characterized in that they produce polysaccharides containing galactose and having molar ratios with respect to this ose of between 0.3 and 0.5 for rhamnose, between 0.4 and 0.6 for mannose, between 1 and 1.8 for glucose, between 0.4 and 0.6 for glucuronic acid, and between 0.1 and 0.3 for galacturonic acid.

9. Strain of bacteria according to claim 8, characterized in that it has been deposited at the CNCM under the n° I-1284.

10. Bacteria according to claim 1, characterized in that they produce polysaccharides containing galactose and having molar ratios with respect to this ose of between 0.2 and 0.3 for rhamnose, between 0.2 and 0.4 for mannose, between 0.5 and 1.1 for glucose, between 0.1 and 0.3 for glucuronic acid, and between 0. 1 and 0.3 for galacturonic acid.

11. Strain of bacteria according to claim 10, characterized in that it has been deposited at the CNCM under the n° I-1285.

12. Bacteria according to claim 1, characterized in that they produce polysaccharides containing galactose and having molar ratios with respect to this ose of between 0.9 and 1.1 for mannose. and between 0.9 and 1.1 for glucose.

13. Strain of bacteria according to claim 12, characterized in that it has been deposited at the CNCM under the n° I-1286.

14. Polysaccharide able to be isolated from bacteria or bacterial strains according to any one of claims 1 to 13, characterized in that it contains galactose and has molar ratios with respect to this ose of between 0.6 and 1.2 for rhamnose, between 1 and 2 for mannose, between 1.1 and 1.5 for glucose, between 0.4 and 0.6 for glucuronic acid, less than 0.07 for N-acetyl galactosamine, and less than 0.04 for N-acetyl glucosamine.

15. Polysaccharide according to claim 14, characterized in that it is substituted by acetyl and/or pyruvyl groups.

16. Polysaccharide according to one of claims 14 and 15, characterized in that it has a mass of less than 10⁶D.

17. Polysaccharide able to be isolated from bacteria or bacterial strains according to any one of claims 1 to 13, characterized in that it contains galactose and has molar ratios with respect to this ose of between 0.2 and 1.4 for rhamnose, between 0.1 and 0.4 for mannose between 3.3 and 10.2 for glucose, and between 0.06 and 0.1 for glucuronic acid.

18. Polysaccharide according to claim 17, characterized in that it is substituted by acetyl groups.

19. Polysaccharide according to one of claims 17 and 18, characterized in that it has a mass of the order of 10⁶D.

20. Polysaccharide able to be isolated from bacteria or bacterial strains according to any one of claims 1 to 13, characterized in that it contains galactose and has molar ratios with respect to this ose of between 0.7 and 0.9 for glucose, between 0.3 and 0.4 for glucuronic acid, and between 0.1 and 0.3 for galacturonic acid.

21. Polysaccharide according to claim 20, characterized in that it contains a hexuronic acid with furan structure substituted on its carbon at position 3 by a lactyl residue.

22. Polysaccharide according to claim 21, characterized in that the molar ratio of the hexuronic acid to galactose is between 0.1 and 0.5.

23. Polysaccharide according to one of claims 21 and 22, characterized in that it has a mass of the order of 4.10⁶D.

24. Polysaccharide according to one of claims 21 to 23, characterized in that the bonds between the oses are formed on the carbon at position 4 of the glucose, the carbons at positions 3, or 4 and 6 of the galactose and the carbons at positions 4 or 3 of the glucuronic acid.

25. Polysaccharide according to one of claims 21 to 24, characterized in that it is branched via a galactose residue with bonds at positions 4 and 6.

26. Polysaccharide according to one of claims 21 to 25, characterized in that it contains repetitions of chains composed of 3 galactoses, 1 glucose, 2 glucuronic acids, 1 galacturonic acid and 1 hexuronic acid with furan structure, substituted on its carbon at position 3 by a lactyl residue.

27. Polysaccharide according to one of claims 20 to 26, characterized in that it is substituted by acetyl groups.

28. Polysaccharide able to be isolated from bacteria or bacterial strains according to any one of claims 1 to 13, characterized in that it contains galactose and has molar ratios with respect to this ose of between 0.2 and 0.3 for rhamnose, between 0.2 and 0.4 for mannose, between 0.5 and 1.1 for glucose, between 0.1 and 0.3 for glucuronic acid, and between 0.1 and 0.3 for galacturonic acid.

29. Polysaccharide according to claim 28, characterized in that it contains a hexuronic acid substituted on its carbon at position 3 by a lactyl residue.

30. Polysaccharide according to one of claims 28 and 29, characterized in that it has a mass of between 5.10⁵ and 2.10⁶D.

31. Polysaccharide able to be isolated from bacteria or bacterial strains according to any one of claims 1 to 13, characterized in that it contains galactose and has molar ratios with respect to this ose of between 0.9 and 1.1 for mannose, and between 0.9 and 1.1 for glucose.

32. Hexose with the following formula: 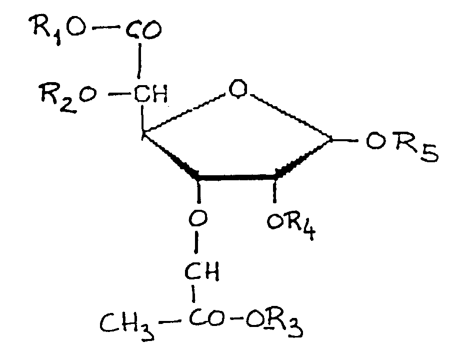 in which R₁ to R₄ each represent a hydrogen atom and OR₅ represents the osidic bond with the other sugars.

33. Hexose according to claim 32 characterized in that it is able to be contained in the polysaccharide according to one of claims 21 and 29.

34. Application of polysaccharides according to any one of claims 14 to 31 for the treatment of media containing metal ions, for example calcium ions or heavy metals (lead, cadmium and mercury) or certain radionuclides (uranium, thorium), the medium to be treated, advantageously in the form of a suspension or a solution, being placed in the presence of an effective quantity of at least one of said polysaccharides, after which the purified medium and the polysaccharide(s) bound to the metal ions to be removed are separated by conventional means.

35. Use of polysaccharides according to any one of claims 14 to 31, as diagnostic agents, in particular by use of their anticoagulant and/or antithrombotic properties.

36. Drug containing a polysaccharide according to one of claims 14 to 31.

37. Use of a polysaccharide according to one of claims 14 to 31 for the manufacture of a drug for the preventive or curative treatment of ailments associated with blood and blood circulation.

38. Process for producing polysaccharides according to one of claims 14 to 31, characterized in that one of the bacteria according to one of claims 1 to 13 is cultured.
